# EUROPEAN PATENT APPLICATION

(11) **EP 1 348 964 A2**
(43) Date of publication of application: **01.10.2003**
(21) Application number: 03007002.3
(22) Date of filing: 27.03.2003
(51) Int. Cl.: G01N 33/68, C12Q 1/68

(54) **Schizophrenia related gene**

(30) Priority: 28.03.2002 EP 02007114
(71) Applicant: Mitsubishi Pharma Corporation, Osaka-shi, Osaka 541-0046 (JP)
(72) Inventor: Cochran, Susan, Yoshitomi Research Institute of, Glasgow G12 8QQ (GB); Yamagami, Keiji, Mitsubishi Pharma Corporation, Tokyo 103-8405 (JP); Ohashi, Yoshitaka, Mitsubishi Pharma Corporation, Tokyo 103-8405 (JP)
(74) Representative: von Kreisler, Alek, Dipl.-Chem.

(57) **Abstract**

The present invention provides a new use of the polynucleotide of SEQ ID NO:1, NO:3 and NO:5 or fragments thereof, or the polypeptide encoded from that polynucleotide in the field of the schizophrenia.

SEQUENCE LISTING

<110> Mitsubishi Pharma Corporation

<120> SCHIZOPHRENIA RELATED GENE

<130> 081081

<150> EP02007114.8
<151> 2002-03-28

<160> 9

<170> PatentIn version 3.0

<210> 1
<211> 3977
<212> DNA
<213> Rattus norvegicus

<220>

<221>  CDS

<222>  (1162)..(2919)

<220>

<221>  gene

<222>  (1)..(3977)

<220>

<221>  polyA_signal

<222>  (3958)..(3963)

<300>

<301>  Luneau,C.J.,  Williams,J.B.,  Marshall,J.,  Levitan,E.S.,  Oliva,C.,
Smith,J.S.,  Antanavage,J.,  Folander,K.,  Stein,R.B.,  Swanson,R.,
Kaczmarek,L.K.  and  Buhrow,S.A.

<302>  Alternate splicing contributes to K+ channel diversity in the
mammalian central nervous system.

<303>  Proc.  Natl.  Acad.  Sci.  USA.

<304>  88

<305>  5

<306>  3932-3936

<308>  NM_012856

<400> 1
gaacgagcaa gcgagcaggc agcggagcag cagcccggag gcagcagcgg cggcggtggt 60
ggcggcagtg gccgctccgc gcctcgcctc cctggcctcg ccagcctcgt tgcgccgcgc 120
ccggacctgc cacccccgcc taccgggccc gacctcggca gcgcccctcg catcggctgc 180
cagcgccacc cgccctccca gagcagtgag cggagtgcgg ggcgcagaaa caccggagcc 240
agtgcccgcc agccccgcca agcaaaacgg gctgagagaa cctacgctcg gcccagcgcg 300
gctccacagc cgtccgggag ctgtcccttc agcaccgccg cggaagcctg agtcccagcg 360
cagcccagcg gaacccctga cccagcccga gcgcaccagg cgctgcgctt agcggcagcc 420
ccgcggggag caccettgtg cccctaccca ggggacctgc catctcctcc gggaggcggg 480
gagaaggaac gaggagggag gtcgggcagc ttcacccgcc accgagggga gtaggcagac 540
aagcaaggag atccgaaggg gatcggtcac gcagggagga gagcaagccg catccgtccc 600
caccgcaccg ggagcgcgct gcggcgacca ccgcagagcc aagcgcggat ttctcgtaac 660
cccctctttc ctagcagggg ctggtctatc ggtccggccc ccgaaacgga gcagctgacc 720
ttcccctaaa aagctttgtg tcgatttctc catttttccc gctgagatgg taccgggagc 780
ccggcccccc catcatctct cccctcctcc cgggcctcgc cgctgagcgc cgccccactc 840
ccttcctccc tccctcttta cctccctcct ctcttccact gcctcctccg cagccaggcc 900
agctccctct cacactaggc gccttaaaga ccctaaggag cccgcgcaca cacccctgga 960
cccgcacctg actgacagct ccccctgagg gggcttggct cgttggttgg ggtgggtggc 1020
cagagccgcg gtcctctgtg ccccccgacg gctgggggga gggggaaga ggccctgcgc 1080
ccccctcccc gtcgccaact ccccctggcg gcagctccca tgggtgtcgc tgggccgcgc 1140
catgcctaag gggcgccgc g atg ggc caa ggg gac gag agc gag cgc atc 1191
Met Gly Gln Gly Asp Glu Ser Glu Arg Ile
1               5                   10

gtg atc aac gtg ggc ggc acg cgc cac cag acg tac cgc tcg acg ctg 1239
Val Ile Asn Val Gly Gly Thr Arg His Gln Thr Tyr Arg Ser Thr Leu
                15                  20                  25

cgc acg ctg ccc ggc acg cgg ctt gcc tgg ctg gcg gag ccg gac gcc 1287
Arg Thr Leu Pro Gly Thr Arg Leu Ala Trp Leu Ala Glu Pro Asp Ala
            30                  35                  40

cac agc cac ttc gac tat gac ccg cgc gct gac gag ttc ttc ttc gac 1335
His Ser His Phe Asp Tyr Asp Pro Arg Ala Asp Glu Phe Phe Phe Asp
        45                  50                  55

cgc cac ccg ggc gtc ttc gct cac atc ctg aac tat tac cgc acc ggc 1383
Arg His Pro Gly Val Phe Ala His Ile Leu Asn Tyr Tyr Arg Thr Gly
    60                  65                  70

aag cta cac tgc ccg gcc gac gtg tgc ggg ccg ctc tac gag gag gag 1431
Lys Leu His Cys Pro Ala Asp Val Cys Gly Pro Leu Tyr Glu Glu Glu
75                  80                  85                  90

ctg gcc ttc tgg ggc atc gac gag acg gac gtg gag ccc tgc tgc tgg 1479
Leu Ala Phe Trp Gly Ile Asp Glu Thr Asp Val Glu Pro Cys Cys Trp
                95                  100                 105

atg acg tat cgc cag cac cgc gac gct gag gag gcg ctg gac agc ttt 1527
Met Thr Tyr Arg Gln His Arg Asp Ala Glu Glu Ala Leu Asp Ser Phe
            110                 115                 120

ggt ggc gcg ccc ttg gac aac agc gcc gac gac gcg gac gcc gac ggc 1575
Gly Gly Ala Pro Leu Asp Asn Ser Ala Asp Asp Ala Asp Ala Asp Gly
        125                 130                 135

ccc ggc gac tcg ggc gac ggc gag gac gag ctg gag atg acc aag aga 1623
Pro Gly Asp Ser Gly Asp Gly Glu Asp Glu Leu Glu Met Thr Lys Arg
    140                 145                 150

ttg gcg ctc agt gac tcc cca gat ggc cgg cct ggc ggc ttc tgg cgc 1671
Leu Ala Leu Ser Asp Ser Pro Asp Gly Arg Pro Gly Gly Phe Trp Arg
155                 160                 165                 170

cgc tgg cag cca cgc atc tgg gca ctg ttc gag gac ccc tac tca tcc 1719

Arg Trp Gln Pro Arg Ile Trp Ala Leu Phe Glu Asp Pro Tyr Ser Ser
                175                 180                 185

cgc tat gcg cgg tac gtg gcc ttt gcc tcc ctc ttc ttc atc ctg gtc 1767
Arg Tyr Ala Arg Tyr Val Ala Phe Ala Ser Leu Phe Phe Ile Leu Val
            190                 195                 200

tcc atc aca acc ttc tgt ctg gag acc cac gag cgc ttc aac ccc atc 1815
Ser Ile Thr Thr Phe Cys Leu Glu Thr His Glu Arg Phe Asn Pro Ile
        205                 210                 215

gtg aac aag aca gaa att gag aac gtt cga aat ggc aca caa gtg cgg 1863
Val Asn Lys Thr Glu Ile Glu Asn Val Arg Asn Gly Thr Gln Val Arg
    220                 225                 230

tac tac cgg gaa gcg gag acg gag gcc ttc ctc acc tac atc gag ggt 1911
Tyr Tyr Arg Glu Ala Glu Thr Glu Ala Phe Leu Thr Tyr Ile Glu Gly
235                 240                 245                 250

gtc tgc gtg gtc tgg ttc acc ttc gag ttc ctc atg cgt gtt gtc ttc 1959
Val Cys Val Val Trp Phe Thr Phe Glu Phe Leu Met Arg Val Val Phe
                255                 260                 265

tgt ccc aac aag gtg gag ttc atc aag aac tcc ctc aat atc att gac 2007
Cys Pro Asn Lys Val Glu Phe Ile Lys Asn Ser Leu Asn Ile Ile Asp
            270                 275                 280

ttc gtg gcc atc ctc ccc ttc tac ctg gag gtg ggc cta agt ggc ctg 2055
Phe Val Ala Ile Leu Pro Phe Tyr Leu Glu Val Gly Leu Ser Gly Leu
        285                 290                 295

tcc tcc aaa gct gcc aag gac gtg cta ggc ttc ctg cgc gtc gtg cgc 2103
Ser Ser Lys Ala Ala Lys Asp Val Leu Gly Phe Leu Arg Val Val Arg
    300                 305                 310

ttc gtg cgc atc ctc cgt atc ttc aag ctg acc cgc cat ttt gtg ggc 2151
Phe Val Arg Ile Leu Arg Ile Phe Lys Leu Thr Arg His Phe Val Gly
315                 320                 325                 330

ctg cgg gtc ctg ggc cac acg ctc cgt gcc agc acc aac gag ttc ctg 2199
Leu Arg Val Leu Gly His Thr Leu Arg Ala Ser Thr Asn Glu Phe Leu
                335                 340                 345

ctg ctt atc atc ttc ctg gcc ctg ggt gtg ctc atc ttt gcc acc atg 2247
Leu Leu Ile Ile Phe Leu Ala Leu Gly Val Leu Ile Phe Ala Thr Met
            350                 355                 360

atc tac tat gct gag agg ata ggg gca cag ccc aat gac ccc agc gcc 2295
Ile Tyr Tyr Ala Glu Arg Ile Gly Ala Gln Pro Asn Asp Pro Ser Ala
        365                 370                 375

agc gaa cac aca cac ttt aaa aat atc ccc atc ggc ttc tgg tgg gct 2343
Ser Glu His Thr His Phe Lys Asn Ile Pro Ile Gly Phe Trp Trp Ala
    380                 385                 390

gtg gtc acc atg acg aca ctg ggc tat gga gac atg tat cct cag acg 2391
Val Val Thr Met Thr Thr Leu Gly Tyr Gly Asp Met Tyr Pro Gln Thr
395                 400                 405                 410

tgg tct ggg atg ttg gtg gga gca ctg tgc gcc ctg gct ggt gtg ctg 2439
Trp Ser Gly Met Leu Val Gly Ala Leu Cys Ala Leu Ala Gly Val Leu
                415                 420                 425

acc att gcc atg ccg gtg ccc gtc atc gtg aac aat ttt ggg atg tac 2487
Thr Ile Ala Met Pro Val Pro Val Ile Val Asn Asn Phe Gly Met Tyr
            430                 435                 440

tac tct tta gcc atg gct aag cag aaa cta cca aag aaa aaa aag aag 2535
Tyr Ser Leu Ala Met Ala Lys Gln Lys Leu Pro Lys Lys Lys Lys Lys
        445                 450                 455

cat att ccg cgg cca cca cag ctg gga tct ccc aat tat tgt aaa tct 2583
His Ile Pro Arg Pro Pro Gln Leu Gly Ser Pro Asn Tyr Cys Lys Ser
    460                 465                 470

gtc gta aac tct cca cac cac agt act cag agt gac aca tgt ccg ctg 2631
Val Val Asn Ser Pro His His Ser Thr Gln Ser Asp Thr Cys Pro Leu
475                 480                 485                 490

gcc cag gaa gaa att tta gaa att aac aga gca gat tcc aaa ctg aat 2679
Ala Gln Glu Glu Ile Leu Glu Ile Asn Arg Ala Asp Ser Lys Leu Asn
                495                 500                 505

ggg gag gtg gcg aag gcc gcg ctg gcg aac gaa gac tgc ccc cac ata 2727

Gly Glu Val Ala Lys Ala Ala Leu Ala Asn Glu Asp Cys Pro His Ile
            510                 515                 520

gac cag gcc ctc act ccc gat gag ggc ctg ccc ttt acc cgc tcg ggc 2775
Asp Gln Ala Leu Thr Pro Asp Glu Gly Leu Pro Phe Thr Arg Ser Gly
        525                 530                 535

acc cgc gag aga tac gga ccc tgc ttc ctc tta tca acc ggg gag tac 2823
Thr Arg Glu Arg Tyr Gly Pro Cys Phe Leu Leu Ser Thr Gly Glu Tyr
    540                 545                 550

gcg tgc ccg cct ggt gga gga atg aga aag gat ctt tgc aaa gaa agc 2871
Ala Cys Pro Pro Gly Gly Gly Met Arg Lys Asp Leu Cys Lys Glu Ser
555                 560                 565                 570

cct gtc att gct aag tat atg ccg aca gag gct gtg aga gtg act tga 2919
Pro Val Ile Ala Lys Tyr Met Pro Thr Glu Ala Val Arg Val Thr
                575                 580                 585

ccaggcggct tggccgagga cactggtggc tattaagcat ctgggtggac ctgcagcccc 2979

tcctcaccct cggacagagt aaattcacgc catgcaggtt tgccggacga gtccgagtgg 3039

ccccgggcat tgtactaaga cggacgtagc tttttctacg gcccacggta actgaccgta 3099

gaggattcgc ccttctttct tttgattttc tttttctttc tttttttttt ttttagaagt 3159

taccttttat ttggggaggg ggggtcaagg ggagccttag catgacttgc atgaagttaa 3219

acagaaaacc cagctaaacc aaccccacca gcccctgctg tgactgtatg ctcactctaa 3279

ccaccaccga gaaccagaga aaggcaagag tctcttattt cttccagcat tctttgacac 3339

gtagtgtcgg agtcaaactg tgacggcgct tggtagaacc ctgtacattt ccgggagtgg 3399

gtgcgggggg gggggggagg tcagggacta aggatggggc caggggagtg gattgctttt 3459

ttgtacacga gacccagagg aaaggtccca aaaaggcagt cagtcatcgg tcacattgac 3519

ctcaccttca tagctcacct ctcgctcgga aaaccaagaa ctatgctccg aataggattg 3579

tggaaattca cactgccacc ctcttccacc tccgtttggc atgcttgcga cccaatggac 3639

ctctccccaa gaccccgaca gcggctagtt taggtcaatt ctctcatctt ggtgtgtagc 3699

tccagtgtga ccaagtttca taacaaattg tttcatagtt acaccacatg gattttccaa 3759

tgtcatttta aagccaggat gtagagtccc tccagttcag gaccacccag aggggcgcct 3819

cccctccacc tttactaagg cgaaaaccga cactgtatgc aatttagtac ttcagagctc 3879

aaagaaaaaa aaaaaaagaa aaaaaaagaa aaaagaaaaa ctgcatgccc aatgttatgc 3939

aaagagattc gagcatgaaa taaattttgt aacatggt 3977

<210> 2

<211> 585

<212> PRT

<213> Rattus norvegicus

<400> 2

Met Gly Gln Gly Asp Glu Ser Glu Arg Ile Val Ile Asn Val Gly Gly
1               5                   10                  15

Thr Arg His Gln Thr Tyr Arg Ser Thr Leu Arg Thr Leu Pro Gly Thr
            20                  25                  30

Arg Leu Ala Trp Leu Ala Glu Pro Asp Ala His Ser His Phe Asp Tyr
        35                  40                  45

Asp Pro Arg Ala Asp Glu Phe Phe Phe Asp Arg His Pro Gly Val Phe
    50                  55                  60

Ala His Ile Leu Asn Tyr Tyr Arg Thr Gly Lys Leu His Cys Pro Ala
65                  70                  75                  80

Asp Val Cys Gly Pro Leu Tyr Glu Glu Glu Leu Ala Phe Trp Gly Ile
                85                  90                  95

Asp Glu Thr Asp Val Glu Pro Cys Cys Trp Met Thr Tyr Arg Gln His
            100                 105                 110

Arg Asp Ala Glu Glu Ala Leu Asp Ser Phe Gly Gly Ala Pro Leu Asp
        115                 120                 125

Asn Ser Ala Asp Asp Ala Asp Ala Asp Gly Pro Gly Asp Ser Gly Asp
    130                 135                 140

Gly Glu Asp Glu Leu Glu Met Thr Lys Arg Leu Ala Leu Ser Asp Ser
145                 150                 155                 160

Pro Asp Gly Arg Pro Gly Gly Phe Trp Arg Arg Trp Gln Pro Arg Ile
                165                 170                 175

Trp Ala Leu Phe Glu Asp Pro Tyr Ser Ser Arg Tyr Ala Arg Tyr Val
            180                 185                 190

Ala Phe Ala Ser Leu Phe Phe Ile Leu Val Ser Ile Thr Thr Phe Cys
        195                 200                 205

Leu Glu Thr His Glu Arg Phe Asn Pro Ile Val Asn Lys Thr Glu Ile
    210                 215                 220

Glu Asn Val Arg Asn Gly Thr Gln Val Arg Tyr Tyr Arg Glu Ala Glu
225                 230                 235                 240

Thr Glu Ala Phe Leu Thr Tyr Ile Glu Gly Val Cys Val Val Trp Phe
                245                 250                 255

Thr Phe Glu Phe Leu Met Arg Val Val Phe Cys Pro Asn Lys Val Glu
            260                 265                 270

Phe Ile Lys Asn Ser Leu Asn Ile Ile Asp Phe Val Ala Ile Leu Pro
        275                 280                 285

Phe Tyr Leu Glu Val Gly Leu Ser Gly Leu Ser Ser Lys Ala Ala Lys
    290                 295                 300

Asp Val Leu Gly Phe Leu Arg Val Val Arg Phe Val Arg Ile Leu Arg
305                 310                 315                 320

Ile Phe Lys Leu Thr Arg His Phe Val Gly Leu Arg Val Leu Gly His
                325                 330                 335

Thr Leu Arg Ala Ser Thr Asn Glu Phe Leu Leu Leu Ile Ile Phe Leu
            340                 345                 350

Ala Leu Gly Val Leu Ile Phe Ala Thr Met Ile Tyr Tyr Ala Glu Arg
        355                 360                 365

Ile Gly Ala Gln Pro Asn Asp Pro Ser Ala Ser Glu His Thr His Phe
    370                 375                 380

Lys Asn Ile Pro Ile Gly Phe Trp Trp Ala Val Val Thr Met Thr Thr
385                 390                 395                 400

Leu Gly Tyr Gly Asp Met Tyr Pro Gln Thr Trp Ser Gly Met Leu Val
                405                 410                 415

Gly Ala Leu Cys Ala Leu Ala Gly Val Leu Thr Ile Ala Met Pro Val
            420                 425                 430

Pro Val Ile Val Asn Asn Phe Gly Met Tyr Tyr Ser Leu Ala Met Ala
        435                 440                 445

Lys Gln Lys Leu Pro Lys Lys Lys Lys Lys His Ile Pro Arg Pro Pro
    450                 455                 460

Gln Leu Gly Ser Pro Asn Tyr Cys Lys Ser Val Val Asn Ser Pro His
465                 470                 475                 480

His Ser Thr Gln Ser Asp Thr Cys Pro Leu Ala Gln Glu Glu Ile Leu
                485                 490                 495

Glu Ile Asn Arg Ala Asp Ser Lys Leu Asn Gly Glu Val Ala Lys Ala
            500                 505                 510

Ala Leu Ala Asn Glu Asp Cys Pro His Ile Asp Gln Ala Leu Thr Pro
        515                 520                 525

Asp Glu Gly Leu Pro Phe Thr Arg Ser Gly Thr Arg Glu Arg Tyr Gly
    530                 535                 540

Pro Cys Phe Leu Leu Ser Thr Gly Glu Tyr Ala Cys Pro Pro Gly Gly
545                 550                 555                 560

Gly Met Arg Lys Asp Leu Cys Lys Glu Ser Pro Val Ile Ala Lys Tyr
                565                 570                 575

Met Pro Thr Glu Ala Val Arg Val Thr
            580                     585

<210>  3

<211>  2441

<212>  DNA

<213>  Rattus norvegicus

<220>

<221>  gene

<222>  (1)..(2411)

<220>

<221>  CDS

<222>  (37)..(1953)

<300>

<301>  McCormack,T., Vega-Saenz de Miera,E.C. and Rudy,B.

<302>  Molecular cloning of a member of a third class of Shaker-family K+
channel genes in mammals

<303>  Proc. Natl. Acad. Sci. U.S.A.

<304>  88

<305>  9

<308>  NM_139217

<400>  3
gtgttcgcct tccctagtca tgtctgagcc acagag atg ggc aag atc gag aac      54
                                        Met Gly Lys Ile Glu Asn
                                        1               5

aac gag agg gtg atc ctc aat gtc gga ggc acc agg cac gaa acc tac      102
Asn Glu Arg Val Ile Leu Asn Val Gly Gly Thr Arg His Glu Thr Tyr
            10                  15                  20

cgc agc act ctc aag acc ctt cct gga act cgc ctg gcc ctt ctc gcc      150
Arg Ser Thr Leu Lys Thr Leu Pro Gly Thr Arg Leu Ala Leu Leu Ala
        25                  30                  35

tcc tct gaa cct cag ggc gac tgc ctg act gct gcg ggt gac aag ctg      198
Ser Ser Glu Pro Gln Gly Asp Cys Leu Thr Ala Ala Gly Asp Lys Leu
    40                  45                  50

cag ccg ctg ccc cct ccg ctg tct cca ccg ccg cga ccg cct ccc ttg      246
Gln Pro Leu Pro Pro Pro Leu Ser Pro Pro Pro Arg Pro Pro Pro Leu
55                  60                  65                  70

tcc cct gtc ccc agc ggc tgc ttc gag ggc ggc gca ggc aac tgc agt      294
Ser Pro Val Pro Ser Gly Cys Phe Glu Gly Gly Ala Gly Asn Cys Ser
                75                  80                  85

tcg cac ggt ggc aat ggc agc gac cac cct ggg gga ggc cgc gaa ttc      342
Ser His Gly Gly Asn Gly Ser Asp His Pro Gly Gly Gly Arg Glu Phe
            90                  95                  100

ttc ttc gat cgc cac cca gga gtc ttc gcc tat gtg ctc aac tac tac      390
Phe Phe Asp Arg His Pro Gly Val Phe Ala Tyr Val Leu Asn Tyr Tyr
        105                 110                 115

cgc acg ggc aag ctg cac tgc ccc gcc gac gtg tgt gga ccg ctc ttc      438
Arg Thr Gly Lys Leu His Cys Pro Ala Asp Val Cys Gly Pro Leu Phe
    120                 125                 130

gag gaa gag ctg gca ttc tgg ggc atc gat gag acc gac gtg gag ccc 486
Glu Glu Glu Leu Ala Phe Trp Gly Ile Asp Glu Thr Asp Val Glu Pro
135                 140                 145                 150

tgc tgc tgg atg acc tac agg cag cac cgg gac gcg gag gag gcc ctg 534
Cys Cys Trp Met Thr Tyr Arg Gln His Arg Asp Ala Glu Glu Ala Leu
                155                 160                 165

gat atc ttc gag aca ccc gac ctc atc gga ggc gac cct ggt gat gat 582
Asp Ile Phe Glu Thr Pro Asp Leu Ile Gly Gly Asp Pro Gly Asp Asp
            170                 175                 180

gag gac cta ggg ggc aag aga ctg ggc att gag gat gct gcg ggg ctg 630
Glu Asp Leu Gly Gly Lys Arg Leu Gly Ile Glu Asp Ala Ala Gly Leu
        185                 190                 195

gga gga ccc gat ggc aag tct ggc cgc tgg agg aag ctg cag cct cgc 678
Gly Gly Pro Asp Gly Lys Ser Gly Arg Trp Arg Lys Leu Gln Pro Arg
    200                 205                 210

atg tgg gct ctc ttt gag gac ccc tat tca tcc aga gcc gct agg ttt 726
Met Trp Ala Leu Phe Glu Asp Pro Tyr Ser Ser Arg Ala Ala Arg Phe
215                 220                 225                 230

att gct ttt gct tct ctg ttc ttc att ttg gtt tcc atc aca acc ttt 774
Ile Ala Phe Ala Ser Leu Phe Phe Ile Leu Val Ser Ile Thr Thr Phe
                235                 240                 245

tgc ctg gag aca cac gaa gct ttc aat att gtt aaa aac aag aca gag 822
Cys Leu Glu Thr His Glu Ala Phe Asn Ile Val Lys Asn Lys Thr Glu
            250                 255                 260

cca gtc atc aac ggc acc agc gct gtt ctc cag tat gaa atc gaa acg 870
Pro Val Ile Asn Gly Thr Ser Ala Val Leu Gln Tyr Glu Ile Glu Thr
        265                 270                 275

gat cct gcc ttg aca tat gtg gaa gga gtg tgt gtg gtg tgg ttt act 918
Asp Pro Ala Leu Thr Tyr Val Glu Gly Val Cys Val Val Trp Phe Thr
    280                 285                 290

ttt gaa ttt tta gtc cgt att gtt ttc tcg ccc aat aaa ctt gag ttc 966
Phe Glu Phe Leu Val Arg Ile Val Phe Ser Pro Asn Lys Leu Glu Phe

295                 300                 305                 310

atc aaa aat cta ttg aac atc att gac ttt gtg gcc atc ctc ccc ttc     1014
Ile Lys Asn Leu Leu Asn Ile Ile Asp Phe Val Ala Ile Leu Pro Phe
                315                 320                 325

tac tta gag gtg gga ctc agc ggg ctg tct tcc aaa gcg gct aaa gat     1062
Tyr Leu Glu Val Gly Leu Ser Gly Leu Ser Ser Lys Ala Ala Lys Asp
            330                 335                 340

gtg ctc ggc ttt ctc agg gtg gtt agg ttt gtg agg atc ctg aga atc     1110
Val Leu Gly Phe Leu Arg Val Val Arg Phe Val Arg Ile Leu Arg Ile
        345                 350                 355

ttc aag ctt acc cgc cat ttc gta ggt ctg aga gtg ctc gga cac act     1158
Phe Lys Leu Thr Arg His Phe Val Gly Leu Arg Val Leu Gly His Thr
    360                 365                 370

ctt cgt gcg agc acc aat gaa ttt ttg ttg ctg atc atc ttt ctg gct     1206
Leu Arg Ala Ser Thr Asn Glu Phe Leu Leu Leu Ile Ile Phe Leu Ala
375                 380                 385                 390

ctg gga gtt ttg ata ttc gct acg atg atc tac tac gct gag cga gta     1254
Leu Gly Val Leu Ile Phe Ala Thr Met Ile Tyr Tyr Ala Glu Arg Val
                395                 400                 405

ggg gct caa cct aat gat ccc tca gcg agt gag cac aca cag ttc aaa     1302
Gly Ala Gln Pro Asn Asp Pro Ser Ala Ser Glu His Thr Gln Phe Lys
            410                 415                 420

aac atc ccc att ggt ttc tgg tgg gct gtg gtg acc atg act acc tta     1350
Asn Ile Pro Ile Gly Phe Trp Trp Ala Val Val Thr Met Thr Thr Leu
        425                 430                 435

ggc tat ggg gat atg tac ccc caa aca tgg tca ggg atg ttg gtg ggg     1398
Gly Tyr Gly Asp Met Tyr Pro Gln Thr Trp Ser Gly Met Leu Val Gly
    440                 445                 450

gcc ttg tgt gct ctg gct gga gtg ctg acc ata gct atg cct gtg ccc     1446
Ala Leu Cys Ala Leu Ala Gly Val Leu Thr Ile Ala Met Pro Val Pro
455                 460                 465                 470

gtc att gtc aac aat ttt ggg atg tac tac tcc ttg gca atg gcg aag 1494
Val Ile Val Asn Asn Phe Gly Met Tyr Tyr Ser Leu Ala Met Ala Lys
                475                 480                 485

cag aaa ctt cca aga aaa aga aag aag cac att cct cct gcc cct ctg 1542
Gln Lys Leu Pro Arg Lys Arg Lys Lys His Ile Pro Pro Ala Pro Leu
            490                 495                 500

gca agc tca cct aca ttt tgc aag aca gaa tta aac atg gct tgt aac 1590
Ala Ser Ser Pro Thr Phe Cys Lys Thr Glu Leu Asn Met Ala Cys Asn
        505                 510                 515

agt acc cag agt gac aca tgt ctg ggc aaa gaa aac cgg ctt ctg gaa 1638
Ser Thr Gln Ser Asp Thr Cys Leu Gly Lys Glu Asn Arg Leu Leu Glu
    520                 525                 530

cat aac aga tca gtg tta tca ggt gac gac agt aca gga agt gag ccg 1686
His Asn Arg Ser Val Leu Ser Gly Asp Asp Ser Thr Gly Ser Glu Pro
535                 540                 545                 550

cca tta tca cct ccg gaa agg ctc ccc atc aga cgc tct agt acc aga 1734
Pro Leu Ser Pro Pro Glu Arg Leu Pro Ile Arg Arg Ser Ser Thr Arg
                555                 560                 565

gac aaa aac aga aga ggg gaa aca tgt ttc ctg ttg acg aca ggt gat 1782
Asp Lys Asn Arg Arg Gly Glu Thr Cys Phe Leu Leu Thr Thr Gly Asp
            570                 575                 580

tac acg tgc gct tct gat gga gga atc agg aaa gga tat gaa aaa tcc 1830
Tyr Thr Cys Ala Ser Asp Gly Gly Ile Arg Lys Gly Tyr Glu Lys Ser
        585                 590                 595

cga agc tta aac aac ata gcg ggc ttg gca ggc aat gct ctg aga ctc 1878
Arg Ser Leu Asn Asn Ile Ala Gly Leu Ala Gly Asn Ala Leu Arg Leu
    600                 605                 610

tct cca gta aca tcc ccc tac aac tct ccg tgt cct ctg agg cgc tct 1926
Ser Pro Val Thr Ser Pro Tyr Asn Ser Pro Cys Pro Leu Arg Arg Ser
615                 620                 625                 630

cgg tct ccc atc cca tct atc ttg taa accaaactgc accaatcggc 1973
Arg Ser Pro Ile Pro Ser Ile Leu

                635

taaattctat cactacaact cctgtccacc ctgtactgga aacaattaac tatagagttt 2033

ctccaggctc cattaagaac agtgtagatc ctgcatgaca ttactattcg aagtcagaaa 2093

tgctactcga gtagctagaa catcttttcc tggctttaaa gatggcctaa agtagtgctg 2153

ctactcagta agagttgccc aatttccttt cgctatacgg tgaccaatag cgtcagatat 2213

tggccagtgc acgaatgcat aagcaaatct tcagggagat attctttaat agtgtgcttc 2273

taaatgccaa ccacttcact ggaccttcct ttcttgtgac tgactccaac ccattctgaa 2333

gatgtctggg atcctatcta gatcgtataa accatgactt tggtcagctc gtttgcatgg 2393

accatcttgc ctgtatcacc tgaatacagt gtgtagcgtc ctggtacc 2441

<210> 4

<211> 638

<212> PRT

<213> Rattus norvegicus

<400> 4

Met Gly Lys Ile Glu Asn Asn Glu Arg Val Ile Leu Asn Val Gly Gly
1               5                   10                  15

Thr Arg His Glu Thr Tyr Arg Ser Thr Leu Lys Thr Leu Pro Gly Thr
            20                  25                  30

Arg Leu Ala Leu Leu Ala Ser Ser Glu Pro Gln Gly Asp Cys Leu Thr
        35                  40                  45

Ala Ala Gly Asp Lys Leu Gln Pro Leu Pro Pro Pro Leu Ser Pro Pro
    50                  55                  60

Pro Arg Pro Pro Pro Leu Ser Pro Val Pro Ser Gly Cys Phe Glu Gly
65                  70                  75                  80

Gly Ala Gly Asn Cys Ser Ser His Gly Gly Asn Gly Ser Asp His Pro
                85                  90                  95

Gly Gly Gly Arg Glu Phe Phe Phe Asp Arg His Pro Gly Val Phe Ala
            100                 105                 110

Tyr Val Leu Asn Tyr Tyr Arg Thr Gly Lys Leu His Cys Pro Ala Asp
        115                 120                 125

Val Cys Gly Pro Leu Phe Glu Glu Glu Leu Ala Phe Trp Gly Ile Asp
    130                 135                 140

Glu Thr Asp Val Glu Pro Cys Cys Trp Met Thr Tyr Arg Gln His Arg
145                 150                 155                 160

Asp Ala Glu Glu Ala Leu Asp Ile Phe Glu Thr Pro Asp Leu Ile Gly
                165                 170                 175

Gly Asp Pro Gly Asp Asp Glu Asp Leu Gly Gly Lys Arg Leu Gly Ile
            180                 185                 190

Glu Asp Ala Ala Gly Leu Gly Gly Pro Asp Gly Lys Ser Gly Arg Trp
        195                 200                 205

Arg Lys Leu Gln Pro Arg Met Trp Ala Leu Phe Glu Asp Pro Tyr Ser

    210                 215                 220

Ser Arg Ala Ala Arg Phe Ile Ala Phe Ala Ser Leu Phe Phe Ile Leu
225                 230                 235                 240

Val Ser Ile Thr Thr Phe Cys Leu Glu Thr His Glu Ala Phe Asn Ile
                245                 250                 255

Val Lys Asn Lys Thr Glu Pro Val Ile Asn Gly Thr Ser Ala Val Leu
            260                 265                 270

Gln Tyr Glu Ile Glu Thr Asp Pro Ala Leu Thr Tyr Val Glu Gly Val
        275                 280                 285

Cys Val Val Trp Phe Thr Phe Glu Phe Leu Val Arg Ile Val Phe Ser
    290                 295                 300

Pro Asn Lys Leu Glu Phe Ile Lys Asn Leu Leu Asn Ile Ile Asp Phe
305                 310                 315                 320

Val Ala Ile Leu Pro Phe Tyr Leu Glu Val Gly Leu Ser Gly Leu Ser
                325                 330                 335

Ser Lys Ala Ala Lys Asp Val Leu Gly Phe Leu Arg Val Val Arg Phe
            340                 345                 350

Val Arg Ile Leu Arg Ile Phe Lys Leu Thr Arg His Phe Val Gly Leu
        355                 360                 365

Arg Val Leu Gly His Thr Leu Arg Ala Ser Thr Asn Glu Phe Leu Leu
    370                 375                 380

Leu Ile Ile Phe Leu Ala Leu Gly Val Leu Ile Phe Ala Thr Met Ile
385                 390                 395                 400

Tyr Tyr Ala Glu Arg Val Gly Ala Gln Pro Asn Asp Pro Ser Ala Ser
                405                 410                 415

Glu His Thr Gln Phe Lys Asn Ile Pro Ile Gly Phe Trp Trp Ala Val
            420                 425                 430

Val Thr Met Thr Thr Leu Gly Tyr Gly Asp Met Tyr Pro Gln Thr Trp
        435                 440                 445

Ser Gly Met Leu Val Gly Ala Leu Cys Ala Leu Ala Gly Val Leu Thr
    450                 455                 460

Ile Ala Met Pro Val Pro Val Ile Val Asn Asn Phe Gly Met Tyr Tyr
465                 470                 475                 480

Ser Leu Ala Met Ala Lys Gln Lys Leu Pro Arg Lys Arg Lys Lys His
                485                 490                 495

Ile Pro Pro Ala Pro Leu Ala Ser Ser Pro Thr Phe Cys Lys Thr Glu
            500                 505                 510

Leu Asn Met Ala Cys Asn Ser Thr Gln Ser Asp Thr Cys Leu Gly Lys
        515                 520                 525

Glu Asn Arg Leu Leu Glu His Asn Arg Ser Val Leu Ser Gly Asp Asp
    530                 535                 540

Ser Thr Gly Ser Glu Pro Pro Leu Ser Pro Pro Glu Arg Leu Pro Ile

545                 550                 555                 560

Arg Arg Ser Ser Thr Arg Asp Lys Asn Arg Arg Gly Glu Thr Cys Phe
                565                 570                 575

Leu Leu Thr Thr Gly Asp Tyr Thr Cys Ala Ser Asp Gly Gly Ile Arg
            580                 585                 590

Lys Gly Tyr Glu Lys Ser Arg Ser Leu Asn Asn Ile Ala Gly Leu Ala
        595                 600                 605

Gly Asn Ala Leu Arg Leu Ser Pro Val Thr Ser Pro Tyr Asn Ser Pro
    610                 615                 620

Cys Pro Leu Arg Arg Ser Arg Ser Pro Ile Pro Ser Ile Leu
625                 630                 635

<210>  5

<211>  3410

<212>  DNA

<213>  Rattus norvegicus

<220>

<221>  CDS

<222>  (223)..(2262)

<220>

<221>  gene

<222>  (1)..(3410)

<300>

<301>  Vega-Saenz de Miera,E., Moreno,H., Fruhling,D., Kentros,C. and
Rudy,B.

<302>  Cloning of ShIII (Shaw-like) cDNAs encoding a novel high-voltage-
activating, TEA-sensitive, type-A K+ channel

<303>  Proc. R. Soc. Lond., B, Biol. Sci.

<304>  248

<305>  1321

<306>  9-18

<308>  NM_053997

<400>  5
aagctttttg tgcaatccca cctcctctag ctaggcttgg ttagagtctg cctagccgtg      60

gccccgccca ctccatcagt ccaatcagct cgtgttctcc ctccacctgc ccaatcactc     120

cctccccatc cattcatcgc ctcctcctgc ccaatcacat ctgcttctct accaaggctc     180

cgccccaaca ccctgccccc ctcagccccg cctccctgtc ca atg ctc agc tca        234
                                               Met Leu Ser Ser
                                               1

gtg tgc gtc tgg tcg ttc agc ggg cgc cag ggg acc cgc aag cag cat       282
Val Cys Val Trp Ser Phe Ser Gly Arg Gln Gly Thr Arg Lys Gln His
5                   10                  15                  20

tct cag ccg gcg cca acg ccg cag ccg cct gag tcc tca ccg ccg cct       330

Ser Gln Pro Ala Pro Thr Pro Gln Pro Pro Glu Ser Ser Pro Pro Pro
                25                  30                  35

ctg cta ccg ccg ccg cag cag cag tgc gct cag ccc ggc acc gcc gcc 378
Leu Leu Pro Pro Pro Gln Gln Gln Cys Ala Gln Pro Gly Thr Ala Ala
            40                  45                  50

tcc ccg gcg ggt gcc ccg ctt tcc tgt ggg cct ggg ggc cgg cgc gcc 426
Ser Pro Ala Gly Ala Pro Leu Ser Cys Gly Pro Gly Gly Arg Arg Ala
        55                  60                  65

gag cca tgc tcc ggg ctg ccg gcg gtg gcc atg ggg cgg cac ggc ggc 474
Glu Pro Cys Ser Gly Leu Pro Ala Val Ala Met Gly Arg His Gly Gly
    70                  75                  80

ggc ggc ggc gac agc ggt aag atc gtg atc aac gtg ggc ggc gtg cgc 522
Gly Gly Gly Asp Ser Gly Lys Ile Val Ile Asn Val Gly Gly Val Arg
85                  90                  95                  100

cat gag acg tac cgc tcc acg ttg cgc acc cta ccg ggg acc cgg ctg 570
His Glu Thr Tyr Arg Ser Thr Leu Arg Thr Leu Pro Gly Thr Arg Leu
                105                 110                 115

gcc ggg ctg acc gag ccc gag gcg gcc gcg cgc ttt gac tac gac ccg 618
Ala Gly Leu Thr Glu Pro Glu Ala Ala Ala Arg Phe Asp Tyr Asp Pro
            120                 125                 130

ggc acg gac gag ttc ttc ttt gac cgt cac ccg ggc gtc ttc gcc tac 666
Gly Thr Asp Glu Phe Phe Phe Asp Arg His Pro Gly Val Phe Ala Tyr
        135                 140                 145

gtg ctc aac tac tac cgc acc ggc aag ctg cac tgc ccg gcc gac gtg 714
Val Leu Asn Tyr Tyr Arg Thr Gly Lys Leu His Cys Pro Ala Asp Val
    150                 155                 160

tgc ggg ccg ctc ttc gag gag gag ctg ggc ttc tgg ggc ata gac gag 762
Cys Gly Pro Leu Phe Glu Glu Glu Leu Gly Phe Trp Gly Ile Asp Glu
165                 170                 175                 180

acg gac gtg gag gcc tgc tgc tgg atg acc tac cgc cag cac cgt gat 810
Thr Asp Val Glu Ala Cys Cys Trp Met Thr Tyr Arg Gln His Arg Asp
                185                 190                 195

gcc gaa gag gca ctg gac tcc ttc gag gct ccg gac tcc tcg ggc aac 858
Ala Glu Glu Ala Leu Asp Ser Phe Glu Ala Pro Asp Ser Ser Gly Asn
            200                 205                 210

gcc aac gcc aac gcc gga ggc gcc cat gat gcg ggc ctg gac gac gag 906
Ala Asn Ala Asn Ala Gly Gly Ala His Asp Ala Gly Leu Asp Asp Glu
        215                 220                 225

gcg ggc gca gga ggt ggc ggc ctg gac ggg gca ggc ggc gag ctc aag 954
Ala Gly Ala Gly Gly Gly Gly Leu Asp Gly Ala Gly Gly Glu Leu Lys
    230                 235                 240

cgt ctg tgt ttt cag gac gcg ggc gga ggt gcc gga gga cct gcc gga 1002
Arg Leu Cys Phe Gln Asp Ala Gly Gly Gly Ala Gly Gly Pro Ala Gly
245                 250                 255                 260

ggc ccg ggc ggc gcg ggc ggc acg tgg tgg agg cgc tgg cag ccc cgc 1050
Gly Pro Gly Gly Ala Gly Gly Thr Trp Trp Arg Arg Trp Gln Pro Arg
                265                 270                 275

gtg tgg gcg ctt ttt gag gac ccc tac tcg tcg cgg gcc gcc agg tac 1098
Val Trp Ala Leu Phe Glu Asp Pro Tyr Ser Ser Arg Ala Ala Arg Tyr
            280                 285                 290

gtg gcc ttc gcc tcc cta ttc ttt atc ctc atc tcc atc acc acc ttc 1146
Val Ala Phe Ala Ser Leu Phe Phe Ile Leu Ile Ser Ile Thr Thr Phe
        295                 300                 305

tgc ctg gag aca cac gag ggc ttc atc cac atc agc aac aag acg gtg 1194
Cys Leu Glu Thr His Glu Gly Phe Ile His Ile Ser Asn Lys Thr Val
    310                 315                 320

acg cag gcc tct cca atc cct ggg gct ccc ccg gag aat atc acc aat 1242
Thr Gln Ala Ser Pro Ile Pro Gly Ala Pro Pro Glu Asn Ile Thr Asn
325                 330                 335                 340

gtg gag gtg gag acg gaa ccc ttt ttg acc tac gtg gaa ggc gtg tgt 1290
Val Glu Val Glu Thr Glu Pro Phe Leu Thr Tyr Val Glu Gly Val Cys
                345                 350                 355

gtg gtc tgg ttc acc ttt gag ttt ctc atg cgg gtc acc ttc tgc ccg 1338

Val Val Trp Phe Thr Phe Glu Phe Leu Met Arg Val Thr Phe Cys Pro
            360                 365                 370

gat aag gtg gag ttt ctc aaa agc agt ctt aac atc atc gac tgt gta 1386
Asp Lys Val Glu Phe Leu Lys Ser Ser Leu Asn Ile Ile Asp Cys Val
        375                 380                 385

gcc atc ttg ccc ttc tac ttg gaa gtg ggc ctg tca ggt ctc agc tcc 1434
Ala Ile Leu Pro Phe Tyr Leu Glu Val Gly Leu Ser Gly Leu Ser Ser
    390                 395                 400

aaa gct gcc aag gac gtg ctg ggc ttc ctg cgc gtc gtc cgc ttc gtg 1482
Lys Ala Ala Lys Asp Val Leu Gly Phe Leu Arg Val Val Arg Phe Val
405                 410                 415                 420

cgc atc ctc cgt atc ttc aag ctg acc cgc cat ttt gtg ggc ctg cgg 1530
Arg Ile Leu Arg Ile Phe Lys Leu Thr Arg His Phe Val Gly Leu Arg
                425                 430                 435

gtc ctg ggc cac acg ctc cgt gcc agc acc aac gag ttc ttg tta ctc 1578
Val Leu Gly His Thr Leu Arg Ala Ser Thr Asn Glu Phe Leu Leu Leu
            440                 445                 450

att att ttc ctg gct ctg ggg gtc ctc atc ttt gcc acc atg atc tac 1626
Ile Ile Phe Leu Ala Leu Gly Val Leu Ile Phe Ala Thr Met Ile Tyr
        455                 460                 465

tat gcc gag cgc atc ggg gcc gat cct gat gac atc ctg ggc tcc aac 1674
Tyr Ala Glu Arg Ile Gly Ala Asp Pro Asp Asp Ile Leu Gly Ser Asn
    470                 475                 480

cac acc tac ttc aag aac atc ccc atc ggc ttc tgg tgg gct gtg gtc 1722
His Thr Tyr Phe Lys Asn Ile Pro Ile Gly Phe Trp Trp Ala Val Val
485                 490                 495                 500

acc atg acc aca ctg ggc tat ggt gac atg tac ccc aag aca tgg tct 1770
Thr Met Thr Thr Leu Gly Tyr Gly Asp Met Tyr Pro Lys Thr Trp Ser
                505                 510                 515

ggg atg ctg gtc ggg gca ctg tgt gcc ctg gct ggt gtg ctg acc att 1818
Gly Met Leu Val Gly Ala Leu Cys Ala Leu Ala Gly Val Leu Thr Ile
            520                 525                 530

gcc atg ccg gtg ccc gtc att gtc aac aac ttc ggc atg tac tat tca 1866
Ala Met Pro Val Pro Val Ile Val Asn Asn Phe Gly Met Tyr Tyr Ser
        535                 540                 545

ctg gct atg gcc aag cag aaa ttg cca aag aag aaa aac aaa cat atc 1914
Leu Ala Met Ala Lys Gln Lys Leu Pro Lys Lys Lys Asn Lys His Ile
    550                 555                 560

ccc agg ccc ccg cag cct ggc tca ccc aac tac tgc aag ccc gat ccc 1962
Pro Arg Pro Pro Gln Pro Gly Ser Pro Asn Tyr Cys Lys Pro Asp Pro
565                 570                 575                 580

ccg cct ccg ccc cca cca cac ccc cac cac ggc agc ggt ggc atc agc 2010
Pro Pro Pro Pro Pro Pro His Pro His His Gly Ser Gly Gly Ile Ser
                585                 590                 595

cca cca ccg ccc atc acc cct cct tcc atg ggg gtg act gtg gct ggg 2058
Pro Pro Pro Pro Ile Thr Pro Pro Ser Met Gly Val Thr Val Ala Gly
            600                 605                 610

gct tac cca cct gga ccc cac acg cac ccc ggg ctg ctt agg ggt ggt 2106
Ala Tyr Pro Pro Gly Pro His Thr His Pro Gly Leu Leu Arg Gly Gly
        615                 620                 625

gct ggg gga ctg gga att atg gga ttg cct cct ctg cca gcc cct ggt 2154
Ala Gly Gly Leu Gly Ile Met Gly Leu Pro Pro Leu Pro Ala Pro Gly
    630                 635                 640

gag ccc tgc cca ctg gct caa gaa gag gtg att gaa acc aac agg gca 2202
Glu Pro Cys Pro Leu Ala Gln Glu Glu Val Ile Glu Thr Asn Arg Ala
645                 650                 655                 660

ggt gag gct gga gcc agg act gga ggt gtg gga aga cct ggg ggt tgg 2250
Gly Glu Ala Gly Ala Arg Thr Gly Gly Val Gly Arg Pro Gly Gly Trp
                665                 670                 675

ggg tcg ggg tag ggggaggagg cggcagggtt ggaggggatg cagaggcagg 2302
Gly Ser Gly

atttctctta ggttcaggct gagcagagtg gaaggcaaca gggcagacag aagggacaag 2362

tggctagaag gctggagagg aggcacaagg agccacagaa accggattcc gtgtacaaga 2422

gagtgtgtga acatggattt cagccaggct gctctgaaag gcccctggtc agaagcagca 2482

cagtgagggt ctttggagaa gagactcaga aacccaggtg tgatggtgca tgcctccctt 2542

cctttgaagg tacttggtac ctcacatact tgggagatag aagtaggagg atgacgagtt 2602

caaggctagc ctgggtgaca tgtccctgtc tcaaaaatca tgggagaaaa aaatgaaaga 2662

gaaagaaagg aaagaggttg ggaggggtag gggagaaaga gaaaggcttg gtggcactct 2722

cctatagttc caacactggg gagactgagg caggagggtc aacacgtgtt ttgaggctat 2782

cctgggctac atacacagaa acaaaaggtc tgagtagctt cctgggggct gctagttcac 2842

accgattctt agggatattc ctatttctct atgagaaggc aagggaagac agggagagat 2902

agaaaggaaa aagggaaagg aatgagagcc acgtatggta atcctaaaat ggaggaggat 2962

caggagttca aggccggtgt cagctacgtg agaccctgtc tcagaaagaa aggggctcct 3022

gtaaagcaca ggaagtcgcg cgtgactcct ggcaaggcta gaggcaggtc tggctactgt 3082

cactttgtga ctgtggccgg tagtcctgag agccagagct ggggaatgta agtcacttaa 3142

ggagagaggg agaatggagt caggctgcca ggagggtcga ggtggaccct cgtaaaggaa 3202

ggcaaaaggg aggaacagac caaggccagc atcagggtgc ggagctgcca ggcagggtct 3262

gcacagggca gcaggaggca ctagggatcc cagagagttc ttgtaagaga agaggcagcc 3322

aggactggtt gaacgtacct gttatcccag gatttgggaa gctgaggcag aaggacagtt 3382

aagttcagag ctagcctgga ctatagag 3410

<210> 6

<211> 679

<212>  PRT

<213>  Rattus norvegicus

<400>  6

Met Leu Ser Ser Val Cys Val Trp Ser Phe Ser Gly Arg Gln Gly Thr
1               5                   10                  15

Arg Lys Gln His Ser Gln Pro Ala Pro Thr Pro Gln Pro Pro Glu Ser
            20                  25                  30

Ser Pro Pro Pro Leu Leu Pro Pro Pro Gln Gln Gln Cys Ala Gln Pro
        35                  40                  45

Gly Thr Ala Ala Ser Pro Ala Gly Ala Pro Leu Ser Cys Gly Pro Gly
    50                  55                  60

Gly Arg Arg Ala Glu Pro Cys Ser Gly Leu Pro Ala Val Ala Met Gly
65                  70                  75                  80

Arg His Gly Gly Gly Gly Gly Asp Ser Gly Lys Ile Val Ile Asn Val
                85                  90                  95

Gly Gly Val Arg His Glu Thr Tyr Arg Ser Thr Leu Arg Thr Leu Pro
            100                 105                 110

Gly Thr Arg Leu Ala Gly Leu Thr Glu Pro Glu Ala Ala Ala Arg Phe
        115                 120                 125

Asp Tyr Asp Pro Gly Thr Asp Glu Phe Phe Phe Asp Arg His Pro Gly
    130                 135                 140

Val Phe Ala Tyr Val Leu Asn Tyr Tyr Arg Thr Gly Lys Leu His Cys
145                 150                 155                 160

Pro Ala Asp Val Cys Gly Pro Leu Phe Glu Glu Glu Leu Gly Phe Trp
                165                 170                 175

Gly Ile Asp Glu Thr Asp Val Glu Ala Cys Cys Trp Met Thr Tyr Arg
            180                 185                 190

Gln His Arg Asp Ala Glu Glu Ala Leu Asp Ser Phe Glu Ala Pro Asp
        195                 200                 205

Ser Ser Gly Asn Ala Asn Ala Asn Ala Gly Gly Ala His Asp Ala Gly
    210                 215                 220

Leu Asp Asp Glu Ala Gly Ala Gly Gly Gly Gly Leu Asp Gly Ala Gly
225                 230                 235                 240

Gly Glu Leu Lys Arg Leu Cys Phe Gln Asp Ala Gly Gly Gly Ala Gly
                245                 250                 255

Gly Pro Ala Gly Gly Pro Gly Gly Ala Gly Gly Thr Trp Trp Arg Arg
            260                 265                 270

Trp Gln Pro Arg Val Trp Ala Leu Phe Glu Asp Pro Tyr Ser Ser Arg
        275                 280                 285

Ala Ala Arg Tyr Val Ala Phe Ala Ser Leu Phe Phe Ile Leu Ile Ser
    290                 295                 300

Ile Thr Thr Phe Cys Leu Glu Thr His Glu Gly Phe Ile His Ile Ser
305                 310                 315                 320

Asn Lys Thr Val Thr Gln Ala Ser Pro Ile Pro Gly Ala Pro Pro Glu
                325                 330                 335

Asn Ile Thr Asn Val Glu Val Glu Thr Glu Pro Phe Leu Thr Tyr Val
            340                 345                 350

Glu Gly Val Cys Val Val Trp Phe Thr Phe Glu Phe Leu Met Arg Val
        355                 360                 365

Thr Phe Cys Pro Asp Lys Val Glu Phe Leu Lys Ser Ser Leu Asn Ile
    370                 375                 380

Ile Asp Cys Val Ala Ile Leu Pro Phe Tyr Leu Glu Val Gly Leu Ser
385                 390                 395                 400

Gly Leu Ser Ser Lys Ala Ala Lys Asp Val Leu Gly Phe Leu Arg Val
                405                 410                 415

Val Arg Phe Val Arg Ile Leu Arg Ile Phe Lys Leu Thr Arg His Phe
            420                 425                 430

Val Gly Leu Arg Val Leu Gly His Thr Leu Arg Ala Ser Thr Asn Glu
        435                 440                 445

Phe Leu Leu Leu Ile Ile Phe Leu Ala Leu Gly Val Leu Ile Phe Ala
    450                 455                 460

Thr Met Ile Tyr Tyr Ala Glu Arg Ile Gly Ala Asp Pro Asp Asp Ile
465                 470                 475                 480

Leu Gly Ser Asn His Thr Tyr Phe Lys Asn Ile Pro Ile Gly Phe Trp
                485                 490                 495

Trp Ala Val Val Thr Met Thr Thr Leu Gly Tyr Gly Asp Met Tyr Pro
            500                 505                 510

Lys Thr Trp Ser Gly Met Leu Val Gly Ala Leu Cys Ala Leu Ala Gly
        515                 520                 525

Val Leu Thr Ile Ala Met Pro Val Pro Val Ile Val Asn Asn Phe Gly
    530                 535                 540

Met Tyr Tyr Ser Leu Ala Met Ala Lys Gln Lys Leu Pro Lys Lys Lys
545                 550                 555                 560

Asn Lys His Ile Pro Arg Pro Pro Gln Pro Gly Ser Pro Asn Tyr Cys
                565                 570                 575

Lys Pro Asp Pro Pro Pro Pro Pro Pro Pro His Pro His His Gly Ser
            580                 585                 590

Gly Gly Ile Ser Pro Pro Pro Pro Ile Thr Pro Pro Ser Met Gly Val
        595                 600                 605

Thr Val Ala Gly Ala Tyr Pro Pro Gly Pro His Thr His Pro Gly Leu
    610                 615                 620

Leu Arg Gly Gly Ala Gly Gly Leu Gly Ile Met Gly Leu Pro Pro Leu
625                 630                 635                 640

Pro Ala Pro Gly Glu Pro Cys Pro Leu Ala Gln Glu Glu Val Ile Glu
                645                 650                 655

Thr Asn Arg Ala Gly Glu Ala Gly Ala Arg Thr Gly Gly Val Gly Arg
            660                 665                 670

Pro Gly Gly Trp Gly Ser Gly
        675

<210> 7

<211> 45

<212> DNA

<213> Artificial

<220>

<223> oligonucleotide (45mer) used for in situ hybridization for the Kv
3.1 alpha-subunit mRNA

<400> 7
atagtcgaag tggctgtggg cgtccggctc cgccagccag gcaag 45

<210> 8

<211> 45

<212> DNA

<213> Artificial

<220>

<223>  oligonucleotide (45mer) used for in situ hybridization for Kv3.2
      mRNA

<400>  8
cacatcttta gccgctttgg aagacagccc gctgagtccc acctc                    45

<210>  9

<211>  45

<212>  DNA

<213>  Artificial

<220>

<223>  oligonucleotide (45mer) used for in situ hybridization for Kv3.3
      mRNA

<400>  9
agaatgctgc ttgcgggtcc cctggcgccc gctgaacgac cagac                    45

## Description

### TECHNICAL FIELD OF THE INVENTION

The present invention relates to the gene of the voltage-gated potassium channels Kv3. The present invention also relates to its use in the therapy and/or diagnosis of schizophrenia and to a method of screening for a compound which modulates the expression of the gene or the activity of the voltage-gated potassium channels.

### BACKGROUND OF THE INVENTION

Schizophrenia is a devastating mental illness that affects 1% of the world population, the aetiology of which remains elusive. To date, there is a poor understanding of the genes involved.

One of the goals of modern antipsychotic drug development is to produce a drug which is more effective in ameliorating the negative symptoms and cognitive deficits characteristic of schizophrenia than existing therapies. Although typical and atypical antipsychotic drugs, such as haloperidol and clozapine respectively, are effective in attenuating the positive symptoms, they are ineffective (haloperidol) or minimally effective (clozapine) against the negative symptoms and cognitive dysfunction associated with the disease (Goldberg, T.E. et al. British Journal of Psychiatry, 1993, 162; 43-48). The development of improved antipsychotic drugs that will have superior action against the negative symptoms and cognitive dysfunction has been severely hampered by the lack of knowledge of which genes are involved and/or associated with schizophrenia. Moreover little is known about the genes, or more specifically any alteration of expression or mutation of the genes in a patient suffering from schizophrenia.

The Shaw-related family of voltage-gated potassium channels (also known as Kv3 channels) are encoded by four genes (Kv3.1, Kv3.2, Kv3.3 and Kv3.4). The products of these genes have differential expression within the mammalian brain, with Kv3.1 and Kv3.3 channels being predominantly located within a subset of GABAergic interneurons within the brain, namely parvalbumin-containing interneurons (Weiser et al., The Journal of Neuroscience, March 1994, 14(3); 949-972). The function of Kv3.1 and Kv3.3 channels within these interneurons is to allow fast repolarisation of the cells and thus, contributes to the fast-firing phenotype of these neurons (Du et al. The Journal of Neuroscience, January 15, 1996, 16(2); 506-518, Chow et al., The Journal of Neuroscience, November 1, 1999, 19(21); 9332-9345; Erisir et al., The Journal of Neurophysiology, 1999, 82; 2476-2489). Kv3.2 expression is predominantly (90%) within thalamic relay neurons throughout the dorsal thalamus and also in parvalbumin-containing and other GABAergic (Marinotti) interneurons (Moreno et al., The Journal of Neuroscience, August, 1995,15(8); 5486-5501). Parvalbumin expression has been shown to be altered within the prefrontal cortex in schizophrenia (Beasley and Reynolds, Schizophrenia Research, 1997, 24; 349-355), however Kv3.1, Kv3.2 and Kv3.3 expression has not been investigated in this disease state.

The *Shaw*-related voltage-gated potassium channels are known to have many names (see below).

| Present Nomenclature (Alternate names) | Splice Variants (Alternate names) | Gene Gene |
|---|---|---|
| **Kv3.1** (KShIIIB; NGK2-KV4) | **Kv3.1a** (NGK2; KShIIIB) **Kv3.1** (KV4) | KCNC1 |
| **Kv3.2** (KShIIIA) | **Kv3.2a** (RKShIIIA; KShIIIA.1) **Kv3.2b** (KShIIIA.3) **Kv3.2d** (KShIIIA.2) | KCNC2 |
| **Kv3.3** (KShIIID) | **Kv3.3a** (KShIIID.1) **Kv3.3b** (KShIIID.2) | KCNC3 |

Table adapted from Weiser et al., The Journal of Neuroscience, March 1994, 14(3); 949-972.

In the present specification, the inventors use the name Kv3.1 for the voltage-gated potassium channel 3.1; Kv3.2 for the voltage-gated potassium channel 3.2 and Kv3.3 for the voltage-gated potassium channel 3.3. Alternate splice variants of each of the Kv3 genes given in the table above), with the splice variants for each gene differing only in their intracellular C-terminal sequence (Coetzee et al., Annals of the New York Academy of Sciences, 1999, 868; 233-285.

The human Kv3 genes have also been cloned. Kv3.1 α-subunit: GenBank accession number NM_004976; Localization of a highly conserved human potassium channel gene (NGK2-Kv4; KCNC1) to chromosome 11p15. Reid T; Rudy B; Vega-Saenz de Miera E; Lau D; Ward DC and Sen K. (1993). Genomics 15 (2), 405-411. The Kv3.1a α-subunit displays ∼99% sequence identity to the rat sequence, and there is >90% sequence identity between rat and mouse sequences. Kv3.2: GenBank accession number AY118169. Kv3.3:GenBank accession number NM_004977; Genomic organization, nucleotide sequence and cellular distribution of a Shaw-related potassium channel gene, Kv3.3, and mapping of Kv3.3 and Kv3.4 to human chromosomes 19 and 1. Ghanshani etal., Genomics 12(2) 1992; 190-196).

Chronic treatment with phencyclidine (hereinafter PCP) in the rat provides an animal model of schizophrenia with superior face, construct and predictive validity (WO01/75440). It has been shown to mimic metabolic changes (metabolic hypofunction in the prefrontal cortex, thalamus and auditory structures) that are observed in schizophrenic patients, that can be correlated to both positive and negative symptomology and cognitive deficits associated with the disease, and also neurochemical changes observed in postmortem tissue from schizophrenic patients such as decreased parvalbumin expression and decreased 5HT2A binding. Parvalbumin mRNA was selectively decreased within certain brain regions in the chronic PCP rat model of schizophrenia, the prelimbic region of the prefrontal cortex and the reticular nucleus of the thalamus and this effect was modulated by chronic antipsychotic treatment. Due to the high degree of co-localization between parvalbumin and the Kv3.1 and Kv3.3 channels, the aim was to determine whether Kv3.1 α-subunit expression is similarly altered by the same chronic PCP rat model of schizophrenia. Also, due to the localization of Kv3.2 channels in thalamic relay neurons and GABAergic interneurons, it was also of interest whether Kv3.2 mRNA expression was also altered by the chronic PCP rat model of schizophrenia.

### SUMMARY OF THE INVENTION

The present inventors have found that the Kv3.1 α-subunit gene, or a nucleotide having SEQ ID NO:1; the Kv3.2 gene, or a nucleotide having SEQ ID NO:3; and Kv3.3 gene, or a nucleotide having SEQ ID NO:5 shows altered expression in the brain of the chronic PCP rat model of schizophrenia, and also found that the nucleotides and the protein that these nucleotides encodes are useful for the diagnosis or treatment of schizophrenia. The present invention also provides a method of screening a compound that regulates expression and activity of this nucleotide and/or protein. Accordingly, the present invention provides the following.
(1) Use of at least one kind of polynucleotide selected from the group consisting of a polynucleotide having SEQ ID NO:1, a polynucleotide having SEQ ID NO:3, a polynucleotide having SEQ ID NO:5, a fragment thereof, and a polynucleotide having a sequence complementary to such sequence, for the diagnosis of schizophrenia.
(2) The use of the above-mentioned (1), wherein the at least one kind of polynucleotide is selected from the group consisting of a polynucleotide having SEQ ID NO:1, a polynucleotide having SEQ ID NO:3, a polynucleotide having SEQ ID NO:5 and a polynucleotide having a sequence complementary to such sequence.
(3) The use of the above-mentioned (1), wherein the at least one kind of polynucleotide is a polynucleotide having SEQ ID NO:1.
(4) Use of at least one kind of polypeptide selected from the group consisting of a polypeptide encoded by the polynucleotide of SEQ ID NO:1, a polypeptide encoded by the polynucleotide of SEQ ID NO:3, a polypeptide encoded by the polynucleotide of SEQ ID NO:5, and a fragment thereof, for the diagnosis of schizophrenia.
(5) The use of the above-mentioned (4), wherein the at least one kind of polypeptide is selected from the group consisting of a polypeptide encoded by the polynucleotide of SEQ ID NO:1, a polypeptide encoded by the polynucleotide of SEQ ID NO:3 and a polypeptide encoded by the polynucleotide of SEQ ID NO:5.
(6) The use of the above-mentioned (4), wherein the at least one kind of polypeptide is a polypeptide encoded by the polynucleotide of SEQ ID NO:1.
(7) Use of at least one kind of polynucleotide selected from the group consisting of a polynucleotide having SEQ ID NO:1, a polynucleotide having SEQ ID NO:3, a polynucleotide having SEQ ID NO:5, a fragment thereof, and a polynucleotide having a sequence complementary to such sequence, for identifying a compound modulating the activity of Kv3 channels.
(8) The use of the above-mentioned (7), wherein the least one kind of polynucleotide is selected from the group consisting of a polynucleotide having SEQ ID NO:1, a polynucleotide having SEQ ID NO:3, a polynucleotide having SEQ ID NO:5 and a polynucleotide having a sequence complementary to such sequence.
(9) The use of the above-mentioned (7), wherein the at least one kind of polynucleotide is a polynucleotide having SEQ ID NO:1.
(10) Use of at least one kind of polypeptide selected from the group consisting of a polypeptide encoded by the polynucleotide of SEQ ID NO:1, a polypeptide encoded by the polynucleotide of SEQ ID NO:3, a polypeptide encoded by the polynucleotide of SEQ ID NO:5, and a fragment thereof, for identifying a compound modulating the activity of Kv3 channels.
(11) The use of the above-mentioned (10), wherein the at least one kind of polypeptide is selected from the group consisting of a polypeptide encoded by the polynucleotide of SEQ ID NO:1, a polypeptide encoded by the polynucleotide of SEQ ID NO:3, and a polypeptide encoded by the polynucleotide of SEQ ID NO:5.
(12) The use of the above-mentioned (10), wherein the at least one kind of polypeptide is a polypeptide encoded by the polynucleotide of SEQ ID NO:1.
(13) A method for screening a compound regulating the expression of at least one kind of polynucleotide selected from the group consisting of a polynucleotide having SEQ ID NO:1, a polynucleotide having SEQ ID NO:3 and a polynucleotide having SEQ ID NO:5, which comprises the steps of
   (a) bringing a test compound into contact with a cell (as used herein, the cell is capable of expression of polynucleotide, which is at least one kind of polynucleotide selected from the group consisting of a polynucleotide having SEQ ID NO:1, a polynucleotide having SEQ ID NO:3, a polynucleotide having SEQ ID NO:5 and a fragment thereof, and whose expression is desired to be controlled;
   (b) detecting the expression of polypeptide encoded by the polynucleotide in the cell; and
   (c) determining a compound promoting or suppressing the expression of the polypeptide as compared to a control (administration of vehicle alone).
(14) A method for screening a compound regulating the expression of a polynucleotide having SEQ ID NO:1, which comprises the steps of:
   (a) bringing a test compound into contact with a cell (as used herein, the cell is capable of expression of a polynucleotide having SEQ ID NO:1) ;
   (b) detecting the expression of polypeptide encoded by the polynucleotide in the cell; and
   (c) determining a compound promoting or suppressing the expression of the polypeptide as compared to a control (administration of vehicle alone).
(15) A method for screening a compound regulating the expression of at least one kind of polynucleotide selected from the group consisting of a polynucleotide having SEQ ID NO:1, a polynucleotide having SEQ ID NO:3 and a polynucleotide having SEQ ID NO:5, which comprises the steps of:
   (a) bringing a test compound into contact with a cell (as used herein, the cell is capable of expression of polynucleotide, which is at least one kind of polynucleotide selected from the group consisting of a polynucleotide having SEQ ID NO:1, a polynucleotide having SEQ ID NO:3, and a polynucleotide having SEQ ID NO:5, and whose expression is desired to be controlled);
   (b) detecting the expression of polynucleotide in the cell; and
   (c) determining a compound promoting or suppressing the expression of the polynucleotide as compared to a control (administration of vehicle alone).
(16) A method for screening a compound regulating the expression of a polynucleotide having SEQ ID NO:1, which comprises the steps of:
   (a) bringing a test compound into contact with a cell (as used herein, the cell is capable of expression of polynucleotide having SEQ ID NO:1);
   (b) detecting the expression of polynucleotide in the cell; and
   (c) determining a compound promoting or suppressing the expression of the polynucleotide as compared to a control (administration of vehicle alone).

### BRIEF DESCRIPTION OF THE DRAWING

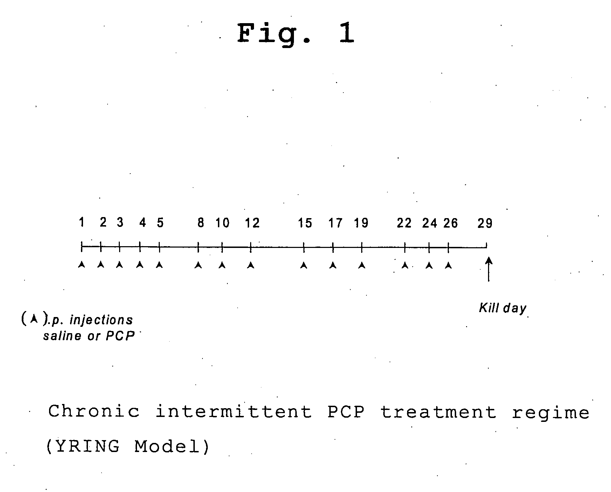

Fig.1 shows a chronic intermittent PCP treatment regime (YRING Model).

### DETAILED DESCRIPTION OF THE INVENTION

The present invention relates to methods for prognostic and/or diagnostic evaluation of schizophrenia and/or for the identification of subjects who are predisposed to schizophrenia, for example by examination of allelic variation of the gene identified herein in an individual or by determination of the expression of the gene in an individual. Furthermore, the invention provides methods for evaluating the efficacy of drugs for such disorders, and monitoring the progress of patient symptoms involved in clinical trials for the treatment of such disorders.

The invention further provides methods for the identification of compounds that modulate the expression of the Kv3 genes and/or the activity of the Kv3.1, Kv3.2 or Kv3.3 channels. Such identified compounds may be used in the treatment of schizophrenia.

By the "regulate" is meant here the action to promote or suppress the expression of the gene, which varies according to the desired effect. In consideration of the application to Schizophrenia, however, screening of a compound that increases the level of expression of Kv3.1 α-subunit gene is preferable and a compound that decreases the level of the Kv3.2 and Kv3.3 gene is preferable.

In the present invention, the "polynucleotide having a sequence complementary" means a sequence complementary to a target sequence to the extent that hybridization under stringent conditions occurs, wherein stringency can be appropriately determined according to the method employed.

In the present invention, the "fragment" means a poly(oligo)nucleotide or a poly(oligo)peptide obtained by fragmentation of polynucleotide having SEQ ID NO:1, a polynucleotide having SEQ ID NO:3, a polynucleotide having SEQ ID NO:5, a polypeptide encoded by the polynucleotide of SEQ ID NO:1 (i.e., SEQ ID NO:2), a polypeptide encoded by the polynucleotide of SEQ ID NO:3 (i.e., SEQ ID NO:4) or a polypeptide encoded by the polynucleotide of SEQ ID NO:5 (i.e., SEQ ID NO:6) to the extent that affords reproduction of characteristic mode of expression found in the pharmacology of schizophrenia. For example, the fragment may be any as long as the expression of a polynucleotide having SEQ ID NO:1, a polynucleotide having SEQ ID NO:3, a polynucleotide having SEQ ID NO:5, a polypeptide having SEQ ID NO:2, a polypeptide having SEQ ID NO:4 and a polypeptide having SEQ ID NO:6 in patients with schizophrenia can be observed and the size thereof is not particularly limited.

The "at least one kind of polynucleotide" of the "at least one kind of polynucleotide selected from the group consisting of a polynucleotide having SEQ ID NO:1, a polynucleotide having SEQ ID NO:3, a polynucleotide having SEQ ID NO:5, a fragment thereof, and a polynucleotide having a sequence complementary to the sequence" intends, in addition to each polynucleotide, two kinds of polynucleotides selected from the group, or 3 kinds of polynucleotides selected from the group and the like. It means, for example, one kind of, two kinds of and all three kinds of polynucleotides selected from the group consisting of a polynucleotide having SEQ ID NO:1, a polynucleotide having SEQ ID NO:3 and a polynucleotide having SEQ ID NO:5. Likewise, the "at least one kind of polypeptide" of the "at least one kind of polypeptide selected from the group consisting of a polypeptide encoded by the polynucleotide of SEQ ID NO:1, a polypeptide encoded by the polynucleotide of SEQ ID NO:3, a polypeptide encoded by the polynucleotide of SEQ ID NO:5, a fragment thereof, and a polypeptide having a sequence complementary to the sequence" intends, in addition to each polypeptide, two kinds of polypeptides selected from the group, or 3 kinds of polypeptides selected from the group and the like. It means, for example, one kind of, two kinds of and all three kinds of polypeptide selected from the group consisting of a polypeptide encoded by the polynucleotide of SEQ ID NO:1, a polypeptide encoded by the polynucleotide of SEQ ID NO:3 and a polypeptide encoded by the polynucleotide of SEQ ID NO:5. Preferably, it is a polynucleotide having SEQ ID NO:1 or polypeptide encoded by the polynucleotide of SEQ ID NO:1.

The present invention provides a method for screening a compound regulating the expression of at least one kind of polynucleotide selected from the group consisting of a polynucleotide having SEQ ID NO:1, a polynucleotide having SEQ ID NO:3 and a polynucleotide having SEQ ID NO:5, which comprises the steps of
(a) bringing a test compound into contact with a cell (as used herein, the cell is capable of expression of polynucleotide, which is at least one kind of polynucleotide selected from the group consisting of a polynucleotide having SEQ ID NO:1, a polynucleotide having SEQ ID NO:3, a polynucleotide having SEQ ID NO:5 and a fragment thereof and whose expression is desired to be controlled) ;
(b) detecting the expression of polypeptide encoded by the polynucleotide in the cell; and
(c) determining a compound promoting or suppressing the expression of the polypeptide as compared to a control (administration of vehicle alone).

In the above-mentioned screening method, the "test compound" is free of any particular limitation as long as it is the object compound for which capability of regulating the expression of at least one kind of polynucleotide selected from the group consisting of a polynucleotide having SEQ ID NO:1, a polynucleotide having SEQ ID NO:3 and a polynucleotide having SEQ ID NO:5, preferably a polynucleotide having SEQ ID NO:1 is desired to be determined, and it may be a novel or known compound. A combination drug can be the object compound. More specifically, chronic PCP administration that affects the expression of the Kv3 mRNAs is combined with administration of the test compound. By analyzing the metabolic and neurochemical state induced by the chronic PCP administration and changes in the expression of the Kv3 mRNAs, the effect of the test compound can be investigated.

In the above-mentioned screening method, the cell is free of any particular limitation as long as it expresses at least one kind of polynucleotide selected from the group consisting of a polynucleotide having SEQ ID NO:1, a polynucleotide having SEQ ID NO:3 and a polynucleotide having SEQ ID NO:5, preferably a polynucleotide having SEQ ID NO:1. It may be naturally occurring or processed to express said polynucleotide by genetic recombination.

For detection of intracellular expression of polypeptide and comparison with control, the methods and techniques generally used in this field can be used and those of ordinary skill in the art can modify them suitably. Specific examples thereof include western blotting capable of detection at a protein level.

Moreover, the method for screening a compound regulating the expression of at least one kind of polynucleotide selected from the group consisting of a polynucleotide having SEQ ID NO:1, a polynucleotide having SEQ ID NO:3 and a polynucleotide having SEQ ID NO:5, may comprise the steps of
(a) bringing a test compound into contact with a cell (as used herein, the cell is capable of expression of polynucleotide, which is at least one kind of polynucleotide selected from the group consisting of a polynucleotide having SEQ ID NO:1, a polynucleotide having SEQ ID NO:3 and a polynucleotide having SEQ ID NO:5 and whose expression is desired to be controlled);
(b) detecting the expression of polynucleotide in the cell; and
(c) determining a compound promoting or suppressing the expression of the polynucleotide as compared to a control (administration of vehicle alone).

Each term is as mentioned above, and for detection of intracellular expression of polynucleotide and comparison with control, the methods and techniques generally used in this field can be used and those of ordinary skill in the art can modify them suitably. Specific examples thereof include northern blotting capable of detection at an mRNA level.

The information obtained through the screening method and diagnostic method of the present invention (hereinafter to be generally referred to as characterization) can suggest relevant methods for the treatment or control of schizophrenia. For example, relevant treatment can include a regulation of gene expression and/or a modulation of gene product activity. Characterization procedures such as those described herein can indicate whether such regulation/modulation should be positive or negative. As used herein, "positive regulation/modulation" refers to an increase in gene expression and/or the activity of gene product of interest. "Negative regulation/modulation", as used herein, refers to a decrease in gene expression and/or activity.

*In vitro* systems can be designed to identify compounds capable of binding with said Kv3 genes products of the invention.

SEQ ID NO:1 shows the nucleotide sequence of the rat Kv3.1 α-subunit gene of the present invention, SEQ ID NO:3 shows the nucleotide sequence of the rat Kv3.2 gene of the present invention and SEQ ID NO:5 shows the nucleotide sequence of the rat Kv3.3 gene of the present invention, which were observed to be differentially expressed in the brain in the chronic PCP rat model of schizophrenia.

SEQ ID NO:2 shows the amino acid sequence of the rat Kv3.1 α-subunit of the present invention, SEQ ID NO:4 shows the amino acid sequence of the rat Kv3.2 of the present invention and SEQ ID NO:6 shows the amino acid sequence of the rat Kv3.3 of the present invention which were observed to be differentially expressed in the brain in the chronic PCP rat model of schizophrenia.

SEQ ID NO:7 shows the oligonucleotide (45mer) used for *in situ* hybridization for the Kv3.1 α-subunit mRNA; SEQ ID NO:8 shows the oligonucleotide (45mer) used for *in situ* hybridization for the Kv3.2 mRNA; SEQ ID NO:9 shows the oligonucleotide (45mer) used for *in situ* hybridization for the Kv3.3 mRNA.

The present invention is explained in more detail in the following by way of Experiments, which are not to be construed as limitative.

In this specification, Y-931 means 8-fluoro-12-(4-methylpiperazin-1-yl)-6H-[1]benzothieno[2,3-b][1,5]benzodiazepine monomaleate, which is known as an atypical antipsychotic drug (WO99/11647).

### Experiment

### Method

### Experiment 1

Male Sprague-Dawley rats, weighing 224-260g at the beginning of the study, were randomly assigned to one of ten treatment groups as follows: vehicle (0.02N HCl in saline)-vehicle (polyethyleneglycol); PCP-vehicle; PCP-Y-931 0.3 mg/kg/day; PCP-Y-931 1 mg/kg/day; PCP-Y-931 3 mg/kg/day; PCP-Y-931 10 mg/kg/day; PCP-olanzapine 0.3 mg/kg/day; PCP-olanzapine 1 mg/kg/day; PCP-olanzapine 3 mg/kg/day; PCP-olanzapine 10 mg/kg/day. The dose of PCP used was 2.24 mg/kg (free base equivalent, equal to 2.58 mg/kg PCP.HCl) and PCP was administered according to the chronic PCP rat model of schizophrenia (WO01/75440). Briefly, i.p. injections of PCP (2.24 mg/kg) or saline (1 ml/kg) were administered once daily on days 1-5, 8,10,12,15,17,19,22,24,26. Osmotic minipumps (2ML4 (28day), Alzet) were implanted subcutaneously on day 8 prior to PCP injection to deliver doses of antipsychotics or vehicle detailed above for 21 days of drug treatment. The osmotic minipumps were primed *in vitro* for 24 hours in order to ensure pumping started immediately upon implantation.

29 days after the first PCP injection, the animals were sacrificed, the brains removed and stored at -70°C. Osmotic minipumps were also removed and the dose administered was verified. The brains were sectioned at -20°C using a cryostat (Leica, CM1850), with 14 µm coronal sections being thaw-mounted onto silanized slides (Dako, Japan) from the following bregma levels according to the Rat Brain Atlas, Paxinos and Watson (2000): 3.22 mm; 1.6 mm, -1.80 mm and -4.8 mm. The sections were then stored at -80°C over silica gel until required. The oligonucleotide (45mer) used for *in situ* hybridization for the Kv3.1 α-subunit mRNA was 5'-ATAGTCGAAGTGGCTGTGGGCGTCCGGCTCCGCCAGCCAGGCAAG-3' (SEQ ID NO:7) complementary to bases 1261-1305 of the rat Kv3.1 α-subunit (GenBank Accession Number NM_012856; Luneau et al., Proc. Natl. Acad. Sci. USA. 1991, 88; 3932-3936). This probe sequence recognizes both alternate splice variants (Kv3.1a and Kv3.1b) of the Kv3.1 channel. The probe was 3' end-labeled with terminal deoxyribonucleotidyl transferase (Boehringer Mannheim) and with the isotope 5-α-³⁵S-dATP (Amersham, specific activity > 1000 Ci/mmol) and incubated at 37°C for one hour. The reaction was terminated by addition of 40 µl diethyl pyrocarbonate (DEPC)-treated water. The probe was purified using the QIAquick nucleotide removal kit (Qiagen). The extent of probe labeling was assessed using β-scintillation counting, and probes labeled from 100,000 to 300,000 d.p.m. µl⁻¹ were used for *in situ* hybridization. On the day of the *in situ* hybridization experiment the sections were fixed in 4% paraformaldehyde-0.1M phosphate buffer for 30 mins at 4°C, rinsed twice for 5 mins in 0.1M phosphate buffer saline (PBS), acetylated with 0.25% acetic anhydride in 0.1M triethanolamine for 10mins, rinsed twice for 5 mins in 2X SSC (0.15M NaCl, 15 mM sodium citrate), dehydrated and delipidated through a graded series of ethanol and chloroform. The sections were then air dried and then hybridized overnight at 42 °C with 0.2-1x10⁻⁶d.p.m. of the labeled probe in 100 µl of hybridization solution (Hybrisol 1, Intergen) containing 0.1M dithiothreitol. After hybridization, the sections were washed twice for 10 secs in 1xSSC at 55°C, four times for 15 mins in 1xSSC at 55°C, for 1 hour in 1xSSC at room temperature and twice in water for 5 mins. The sections were then dipped successively in 60%, 80%, 90%, 95% and 100% ethanol and air dried. The sections were exposed to BAS 5000 image plates and developed after 7 days. Kv3.1 α-subunit mRNA expression was quantified using a computer-based densitometry system (MCID 5, Canada). Statistical analysis of the data was performed using a students t-test (vehicle-vehicle vs PCP-vehicle) followed by Dunnett's Method (PCP-vehicle vs PCP-antipsychotics). Statistical significance was defined as p<0.05.

### Experiment 2

In a second experiment using different method for in situ hybridisation, the effect of chronic PCP treatment on Kv3.1, Kv3.2 and Kv3.3 mRNA were examined. 20 male hooded Long Evans rats (250-300 g at the beginning of the study, Harlan UK) were randomly assigned to either vehicle (saline, n=10) or PCP (2.58 mg/kg PCP.HCl, n=10) treatment groups. Animals were treated according to the YRING model (Fig. 1) and killed by cervical dislocation 72 hours after the last exposure to PCP. The brains were stored at -70°C until required.

### In situ hybridisation

20 µm coronal sections were cut on a cryostat at -20°C and collected onto poly-L-lysine coated slides. The sections were fixed, dehydrated and stored under ethanol at 4°C until required. 45mer oligonucleotide probes designed against rat mRNA for parvalbumin (GeneBank Accession number NM_022499, bases 223-267), Kv3:1 (GeneBank Accession number NM_012856, bases 1261-1305; SEQ ID NO: 7), Kv3.2 (GeneBank Accession number NM_139217, bases 1021-1065; SEQ ID NO: 8) and Kv3.3 (GeneBank Accession number NM 053997, bases 241-285; SEQ ID NO: 9) were 3' end-labeled with terminal deoxyribonucleotidyl transferase and ³⁵Sd-ATP. On the day of *in situ* hybridization, the sections from the chronic study were allowed to dry at room temperature then hybridized with the appropriate probe overnight at 42°C. The sections were then washed, dehydrated and air-dried before being exposed to x-ray film for 7-10 days.

The quantification of mRNA expression was carried out using computer-based densitometry (MCID 5). Bilateral relative optical density measurements were taken from duplicate sections from each animal (n=8-10 per treatment group for the chronic study). The results were analyzed using individual t-tests for each brain region. Statistical significance was defined as p < 0.05, again after correction for multiple comparisons using the sequential Bonferroni correction factor.

### Results

### Experiment 1

Table 1 shows the effect of chronic PCP and antipsychotic treatment on Kv3.1 α-subunit mRNA expression. Chronic PCP treatment selectively decreased Kv3.1 α-subunit mRNA expression within the prelimbic region of the prefrontal cortex (PrL, -31%), the dorsal part of the reticular nucleus of the thalamus (dRt, -15%) and the dorsolateral striatum (DLStr, -16%). Within the PrL, both Y-931 and olanzapine, at all doses administered, significantly reversed the PCP-induced decrease back to control levels. However, within the dRt and the DLStr, neither Y-931 nor olanzapine significantly modulated the PCP-induced effect.

Compared to PCP-vehicle, PCP-olanzapine treatment (1 mg/kg/day, only) produced a significant decrease in Kv3.1 α-subunit mRNA expression within the granule cell layer of the dentate gyrus (DGgcl) and a significant increase in Kv3.1 α-subunit mRNA expression within the ventromedial striatum (VMStr) (0.3 & 3 mg/kg/day olanzapine, only).

### Experiment 2

Table 2 shows the levels of Kv3.1 mRNA expression within the rat brain after chronic intermittent treatment according to the YRING model. Very selective changes in Kv3.1 mRNA expression were observed after chronic intermittent exposure to PCP. A significant decrease in Kv3.1 mRNA expression was observed within the prelimbic cortex (-23%) and within the ventral part of the reticular thalamic nucleus (-19%). No other changes were observed in any other regions.

Table 3 shows the levels of Kv3.2 mRNA expression within the rat brain after chronic intermittent treatment according to the YRING model. A significant increase in Kv3.2 mRNA expression was observed within the prelimbic cortex (+16%) and the primary motor cortex (+13%). A small but significant decrease in Kv3.2 mRNA expression was also observed within the anteromedial thalamic nucleus (-9%). No significant changes were observed after chronic intermittent. PCP treatment within any other regions.

Table 3 shows the levels of Kv3.3 mRNA expression within the rat brain after chronic intermittent treatment according to the YRING model. Within cortical regions, an increase in Kv3.3 mRNA expression after chronic intermittent exposure to PCP was observed within the prelimbic cortex (+18%) and the ventral orbital cortex (+14%). A significant increase in Kv3.3 mRNA expression was also observed within the anterodorsal thalamic nucleus (+21%) and the ventral part of the reticular thalamic nucleus (+33%). No other changes were observed in any other regions.

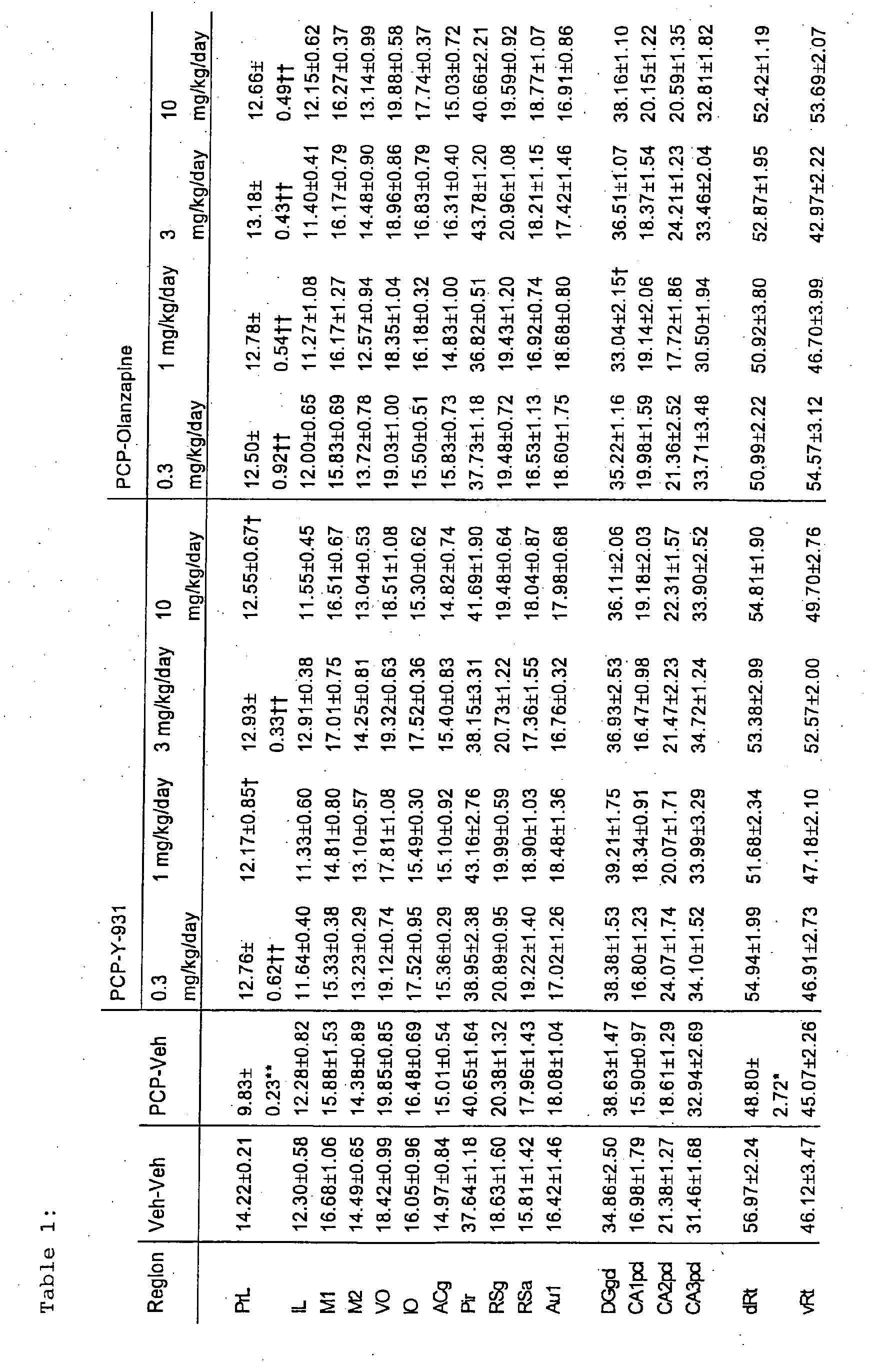

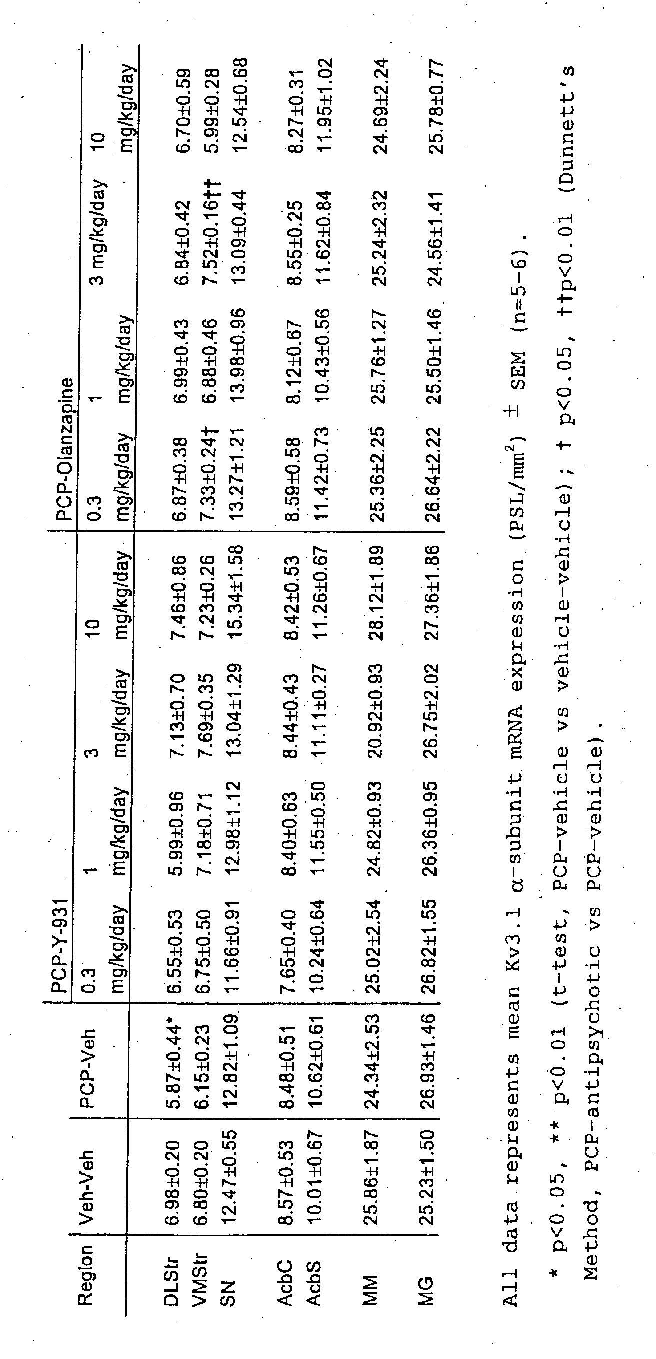

**Table 2:**

| Kv3.1 mRNA expression in rat brain after chronic intermittent exposure to vehicle or PCP (YRING model). Data expressed as mean ± SEM relative optical density (ROD) (n=8-10 per treatment group). ** p < 0.01 compared to vehicle at appropriate time point (student t-test, corrected for multiple comparisons by application of the sequential Bonferroni correction factor). | | | |
|---|---|---|---|
| | Relative Optical Density (ROD) | | |
| Brain Region | Vehicle | PCP (2.58mg/kg) | % Control |
| **Cortical Regions** | | | |
| PrL | 0.112±0.002 | 0.086±0.004** | 77±4** |
| IL | 0.103±0.003 | 0.098±0.004 | 95±4 |
| vO | 0.122±0.003 | 0.122±0.004 | 100±3 |
| lO | 0.101±0.003 | 0.092±0.003 | 91±3 |
| M1 | 0.123±0.004 | 0.120±0.004 | 98±3 |
| M2 | 0.116±0.002 | 0.106±0.004 | 91±3 |
| ACg | 0.126±0.006 | 0.116±0.004 | 92±3 |
| Pir | 0.263±0.006 | 0.243±0.014 | 92±5 |
| RSG | 0.148±0.006 | 0.153±0.008 | 103±5 |
| RSA | 0.127±0.005 | 0.135±0.006 | 106±5 |
| Au1 | 0.138±0.008 | 0.137±0.009 | 99±6 |
| LEnt | 0.096±0.008 | 0.093±0.005 | 97±5 |
| | | | |

| **Thalamus** | | | |
|---|---|---|---|
| AD | 0.215±0.006 | 0.216±0.006 | 100±3 |
| dRt | 0.274±0.007 | 0.258±0.013 | 94±5 |
| vRt | 0.275±0.010 | 0.224±0.008** | 81±3** |
| | | | |

| **Hippocampus** | | | |
|---|---|---|---|
| DG gcl | 0.244±0.005 | 0.239±0.012 | 98±5 |
| CA1pcl | 0.128±0.006 | 0.122±0.007 | 95±5 |
| CA2pcl | 0.156±0.009 | 0.156±0.010 | 100±6 |
| CA3pcl | 0.202±0.007 | 0.203±0.009 | 100±4 |

**Table 3:**

| Kv3.2 mRNA expression in rat brain after chronic intermittent exposure to vehicle or PCP (YRING model). Data expressed as mean ± SEM relative optical density (ROD) (n=8-10 per treatment group). * p < 0.05 compared to vehicle at appropriate time point (student t-test, corrected for multiple comparisons by application of the sequential Bonferroni correction factor). | | | |
|---|---|---|---|
| | Relative Optical Density (ROD) | | |
| Brain Region | Vehicle | PCP (2.58mg/kg) | % Control |
| **Cortical Regions** | | | |
| PrL | 0.079±0.002 | 0.092±0.003* | 116±4 |
| IL | 0.079±0.003 | 0.083±0.002 | 105±2 |
| vO | 0.074±0.002 | 0.078±0.003 | 105±4 |
| lO | 0.069±0.002 | 0.073±0.002 | 106±3 |
| M1 | 0.067±0.002 | 0.076±0.003* | 113±4 |
| M2 | 0.076±0.003 | 0.079±0.004 | 104±5 |
| ACg | 0.078±0.002 | 0.075±0.002 | 96±2 |
| Pir | 0.140±0.005 | 0.138±0.006 | 98±4 |
| RSG | 0.065±0.004 | 0.069±0.002 | 106±3 |
| RSA | 0.070±0.002 | 0.065±0.002 | 93±3 |
| Au1 | 0.070±0.002 | 0.068±0.003 | 97±4 |
| LEnt | 0.051±0.003 | 0.048±0.002 | 94±4 |
| | | | |

| **Thalamus** | | | |
|---|---|---|---|
| AV | 0.275±0.006 | 0.269±0.005 | 98±2 |
| AM | 0.248±0.005 | 0.227±0.006* | 91±2 |
| MD | 0.211±0.008 | 0.187±0.010 | 89±5 |
| VA | 0.240±0.008 | 0.223±0.006 | 93±2 |
| VM | 0.206±0.009 | 0.201±0.005 | 98±2 |
| VL | 0.211±0.010 | 0.208±0.004. | 98±2 |
| LDVL | 0.194±0.009 | 0.197±0.004 | 102±2 |
| LDDM | 0.203±0.008 | 0.205±0.007 | 101±3 |
| VPL | 0.175±0.007 | 0.180±0.005 | 103±3 |
| | | | |
| MGD | 0.175±0.006 | 0.164±0.006 | 94±3 |
| MGV | 0.193±0.007 | 0.185±0.003 | 96±2 |
| DLG | 0.195±0.012 | 0.222±0.007 | 114±4 |
| | | | |

| **Hippocampus** | | | |
|---|---|---|---|
| DG gcl | 0.097±0.004 | 0.095±0.007 | 98±7 |
| CA1pcl | 0.069±0.004 | 0.075±0.004 | 109±6 |
| CA2pcl | 0.080±0.002 | 0.082±0.003 | 102±4 |
| CA3pcl | 0.142±0.005 | 0.134±0.005 | 94±4 |

**Table 4:**

| Kv3.3 mRNA expression in rat brain after chronic intermittent exposure to vehicle or PCP (YRING model). Data expressed as mean ± SEM relative optical density (ROD) (n=8-10 per treatment group). * p < 0.05 compared to vehicle at appropriate time point (student t-test, corrected for multiple comparisons by application of the sequential Bonferroni correction factor). | | | |
|---|---|---|---|
| | Relative Optical Density (ROD) | | |
| Brain Region | Vehicle | PCP (2.58mg/kg) | % Control |
| **Cortical Regions** | | | |
| PrL | 0.057±0.002 | 0.067±0.003* | 118±5 |
| IL | 0.053±0.002 | 0.059±0.002 | 111±4 |
| vO | 0.073±0.003 | 0.083±0.003* | 114±4 |
| lO | 0.053±0.002 | 0.058±0.002 | 109±4 |
| M1 | 0.085±0.003 | 0.093±0.002 | 109±2 |
| M2 | 0.073±0.002 | 0.075±0.003 | 103±4 |
| ACg | 0.068±0.003 | 0.069±0.003 | 101±4 |
| Pir | 0.111±0.005 | 0.115±0.006 | 104±5 |
| RSG | 0.087±0.004 | 0.092±0.004 | 106±4 |
| RSA | 0.066±0.003 | 0.067±0.003 | 102±4 |
| Au1 | 0.075±0.002 | 0.076±0.003 | 101±4 |
| LEnt | 0.042±0.002 | 0.047±0.002 | 112±5 |
| | | | |

| **Thalamus** | | | |
|---|---|---|---|
| AD | 0.173±0.009 | 0.209±0.001* | 121±1 |
| dRt | 0.186±0.001 | 0.198±0.006 | 106±3 |
| vRt | 0.121±0.010 | 0.161±0.007* | 133±6 |
| | | | |

| **Hippocampus** | | | |
|---|---|---|---|
| DG gcl | 0.254±0.009 | 0.275±0.011 | 108±4 |
| CA1 pcl | 0.144±0.006 | 0.146±0.006 | 101±4 |
| CA2 pcl | 0.149±0.005 | 0.148±0.007 | 99±5 |
| CA3 pcl | 0.204±0.006 | 0.210±0.006 | 103±3 |

### Abbreviations:

PrL, prelimbic cortex;
IL, infralimbic cortex;
M1, primary motor cortex;
M2, secondary motor cortex;
vO, ventral orbital cortex;
lO, lateral orbital cortex;
Acg, anterior cingulate cortex;
Pir, piriform cortex;
RSg, granular retrosplenial cortex;
Rsa, agranular retrosplenial cortex;
Au1, primary auditory cortex;
LEnt, lateral entorhinal cortex;
DGgcl, granule cell layer of the dentate gyrus;
CA1-CA3 pcl, pyramidal cell layer of the CA1-CA3 fields of the hippocampus;
AD, anterodorsal nucleus of the thalamus;
AV, anteroventral nucleus of the thalamus;
AM, ateromedial nucleus of the thalamus;
MD, mediodorsal nucleus of the thalamus;
VA, ventral anterior nucleus of the thalamus;
VM, ventral medial nucleus of the thalamus;
VL, ventral lateral nucleus of the thalamus;
LDVL, laterodorsal thalamic nucleus, ventrolateral part;
LDDM, laterodorsal thalamic nucleus, dorsomedial part;
VPL, ventral posterolateral nucleus of the thalamus;
MGD, medial genicluate nucleus, dorsal part;
MGV, medial genicluate nucleus, ventral part;
DLG, dorsal lateral geniculate nucleus
dRt, vRt, dorsal and ventral parts of the reticular nucleus of the thalamus.
DLStr, dorsolateral striatum;
VMStr, ventromedial striatum;
SN, substantia nigra;
AcbC, nucleus accumbens core;
AcbS, nucleus accumbens shell;
MM, mammillary body;
MG, medial geniculates.

The decrease in Kv3.1 α-subunit mRNA expression observed in these studies parallel the selective changes previously observed in parvalbumin mRNA expression within the prelimbic cortex and the reticular nucleus of the thalamus. Moreover, the PCP-induced decreases in Kv3.1 α-subunit mRNA expression were reversed by chronic co-administration of Y-931 and olanzapine, as were PCP-induced changes in parvalbumin mRNA expression. Since parvalbumin expression is altered within the prefrontal cortex of schizophrenic patients and in rats after treatment with chronic PCP, these data suggest that Kv3.1 α-subunit mRNA may also be altered within schizophrenia in a similar manner.

As the chronic model of schizophrenia also produces an increase in Kv3.2 and Kv3.3 mRNA expression in the rat, these data suggest that Kv3.2 and Kv3.3 mRNA may also be altered in schizophrenia.

Drugs acting at the Kv3 channels have potential therapeutic roles in Schizophrenia. The hypothesis is that the decrease in Kv3.1 mRNA may affect the firing activity of the GABAergic interneurons in which it is located, which further contributes to an imbalance between the excitatory (glutamatergic) and inhibitory (GABAergic) pathways within the prefrontal cortex (which may be linked to negative symptoms and cognitive dysfunction). The changes in Kv3.2 mRNA suggest that thalamic relay neurons may also be misfiring, which again may contribute to an imbalance between the excitatory (glutamatergic) and inhibitory (GABAergic) pathways within the prefrontal cortex. The changes in Kv3.3 mRNA may also disrupt the balance between the excitatory (glutamatergic) and inhibitory (GABAergic) pathways within the prefrontal cortex.
A drug which can restore Kv3.1, Kv3.2 and Kv3.3 expression may restore GABAergic interneuron activity back to normal.

Kv3.1 may be a good marker for the screening of antipsychotic activity because of the reversal with the atypical antipsychotic olanzapine and Y-931 as shown in these data.

## Claims

1. Use of at least one kind of polynucleotide selected from the group consisting of a polynucleotide having SEQ ID NO:1, a polynucleotide having SEQ ID NO:3, a polynucleotide having SEQ ID NO:5, a fragment thereof, and a polynucleotide having a sequence complementary to such sequence, for the diagnosis of schizophrenia.

2. The use of claim 1, wherein the at least one kind of polynucleotide is selected from the group consisting of a polynucleotide having SEQ ID NO:1, a polynucleotide having SEQ ID NO:3, a polynucleotide having SEQ ID NO:5 and a polynucleotide having a sequence complementary to such sequence.

3. The use of claim 1, wherein the at least one kind of polynucleotide is a polynucleotide having SEQ ID NO:1.

4. Use of at least one kind of polypeptide selected from the group consisting of a polypeptide encoded by the polynucleotide of SEQ ID NO:1, a polypeptide encoded by the polynucleotide of SEQ ID NO:3, a polypeptide encoded by the polynucleotide of SEQ ID NO:5, and a fragment thereof, for the diagnosis of schizophrenia.

5. The use of claim 4, wherein the at least one kind of polypeptide is selected from the group consisting of a polypeptide encoded by the polynucleotide of SEQ ID NO:1, a polypeptide encoded by the polynucleotide of SEQ ID NO:3 and a polypeptide encoded by the polynucleotide of SEQ ID NO:5.

6. The use of claim 4, wherein the at least one kind of polypeptide is a polypeptide encoded by the polynucleotide of SEQ ID NO:1.

7. Use of at least one kind of polynucleotide selected from the group consisting of a polynucleotide having SEQ ID NO:1, a polynucleotide having SEQ ID NO:3, a polynucleotide having SEQ ID NO:5, a fragment thereof, and a polynucleotide having a sequence complementary to such sequence, for identifying a compound modulating the activity of Kv3 channels.

8. The use of claim 7, wherein the least one kind of polynucleotide is selected from the group consisting of a polynucleotide having SEQ ID NO:1, a polynucleotide having SEQ ID NO:3, a polynucleotide having SEQ ID NO:5 and a polynucleotide having a sequence complementary to such sequence.

9. The use of claim 7, wherein the at least one kind of polynucleotide is a polynucleotide having SEQ ID NO:1.

10. Use of at least one kind of polypeptide selected from the group consisting of a polypeptide encoded by the polynucleotide of SEQ ID NO:1, a polypeptide encoded by the polynucleotide of SEQ ID NO:3, a polypeptide encoded by the polynucleotide of SEQ ID NO:5, and a fragment thereof, for identifying a compound modulating the activity of Kv3 channels.

11. The use of the claim 10, wherein the at least one kind of polypeptide is selected from the group consisting of a polypeptide encoded by the polynucleotide of SEQ ID NO:1, a polypeptide encoded by the polynucleotide of SEQ ID NO:3, and a polypeptide encoded by the polynucleotide of SEQ ID NO:5.

12. The use of claim 10, wherein the at least one kind of polypeptide is a polypeptide encoded by the polynucleotide of SEQ ID NO:1.

13. A method for screening a compound regulating the expression of at least one kind of polynucleotide selected from the group consisting of a polynucleotide having SEQ ID NO:1, a polynucleotide having SEQ ID NO:3 and a polynucleotide having SEQ ID NO:5, which comprises the steps of
(a) bringing a test compound into contact with a cell (as used herein, the cell is capable of expression of polynucleotide, which is at least one kind of polynucleotide selected from the group consisting of a polynucleotide having SEQ ID NO:1, a polynucleotide having SEQ ID NO:3, a polynucleotide having SEQ ID NO:5 and a fragment thereof, and whose expression is desired to be controlled;
(b) detecting the expression of polypeptide encoded by the polynucleotide in the cell; and
(c) determining a compound promoting or suppressing the expression of the polypeptide as compared to a control (administration of vehicle alone).

14. A method for screening a compound regulating the expression of a polynucleotide having SEQ ID NO:1, which comprises the steps of:
(a) bringing a test compound into contact with a cell (as used herein, the cell is capable of expression of a polynucleotide having SEQ ID NO:1);
(b) detecting the expression of polypeptide encoded by the polynucleotide in the cell; and
(c) determining a compound promoting or suppressing the expression of the polypeptide as compared to a control (administration of vehicle alone).

15. A method for screening a compound regulating the expression of at least one kind of polynucleotide selected from the group consisting of a polynucleotide having SEQ ID NO:1, a polynucleotide having SEQ ID NO:3 and a polynucleotide having SEQ ID NO:5, which comprises the steps of:
(a) bringing a test compound into contact with a cell (as used herein, the cell is capable of expression of polynucleotide, which is at least one kind of polynucleotide selected from the group consisting of a polynucleotide having SEQ ID NO:1, a polynucleotide having SEQ ID NO:3, and a polynucleotide having SEQ ID NO:5, and whose expression is desired to be controlled);
(b) detecting the expression of polynucleotide in the cell; and
(c) determining a compound promoting or suppressing the expression of the polynucleotide as compared to a control (administration of vehicle alone).

16. A method for screening a compound regulating the expression of a polynucleotide having SEQ ID NO:1, which comprises the steps of:
(a) bringing a test compound into contact with a cell (as used herein, the cell is capable of expression of polynucleotide having SEQ ID NO:1) ;
(b) detecting the expression of polynucleotide in the cell; and
(c) determining a compound promoting or suppressing the expression of the polynucleotide as compared to a control (administration of vehicle alone).
